# EUROPEAN PATENT SPECIFICATION

(11) **EP 3 555 305 B1**
(45) Date of publication and mention of the grant of the patent: **17.02.2021**
(21) Application number: 17715473.9
(22) Date of filing: 04.04.2017
(51) Int. Cl.: C12Q 1/68

(54) **METHOD FOR INCREASING THROUGHPUT OF SINGLE MOLECULE SEQUENCING BY CONCATENATING SHORT DNA FRAGMENTS**
VERFAHREN ZUR ERHÖHUNG DES DURCHSATZES VON EINZELMOLEKÜLSEQUENZIERUNG DURCH VERKNÜPFUNG KURZER DNA-FRAGMENTE
PROCÉDÉ POUR AUGMENTER LE DÉBIT D'UN SÉQUENÇAGE DE MOLÉCULE UNIQUE PAR CONCATÉNATION DE FRAGMENTS D'ADN COURT

(30) Priority: 16.12.2016 US 201662435517 P; 22.03.2017 US 201762475148 P; 03.04.2017 US 201762481035 P
(43) Date of publication of application: 23.10.2019
(73) Proprietor: F. Hoffmann-La Roche AG, 4070 Basel (CH); Roche Diagnostics GmbH, 68305 Mannheim (DE)
(72) Inventor: SCHLECHT, Ulrich, Sunnyvale, California 94806 (US); MOK, Janine, Palo Alto, California 94303 (US); SO, Austin, Pleasanton, California 95050 (US)
(74) Representative: Hildebrandt, Martin K. E.
(86) International application number: PCT/EP2017/057975
(87) International publication number: WO 2018/108328

(56) References cited:
- WO-A1-2012/012037
- WO-A1-2013/096802
- WO-A1-2016/063034
- WO-A2-2013/040060
- M. J. FULLWOOD ET AL: "Next-generation DNA sequencing of paired-end tags (PET) for transcriptome and genome analyses", GENOME RESEARCH, vol. 19, no. 4, 1 April 2009 (2009-04-01), pages 521-532, XP055015048, ISSN: 1088-9051, DOI: 10.1101/gr.074906.107
- SEAN C. SLEIGHT ET AL: "Randomized BioBrick Assembly: A Novel DNA Assembly Method for Randomizing and Optimizing Genetic Circuits and Metabolic Pathways", ACS SYNTHETIC BIOLOGY, vol. 2, no. 9, 10 July 2013 (2013-07-10), pages 506-518, XP055400088, Washington, DC,USA ISSN: 2161-5063, DOI: 10.1021/sb4000542 & Sean C Sleight ET AL: "Supplementary Info", ACS Synth. Biol., 10 July 2013 (2013-07-10), XP055400084, Retrieved from the Internet: URL:http://pubs.acs.org/doi/suppl/10.1021/ sb4000542/suppl_file/sb4000542_si_001.pdf [retrieved on 2017-08-22]

## Description

### FIELD OF THE INVENTION

The invention relates to the field of nucleic acid sequencing. More specifically, the invention relates to the field of creating libraries of nucleic acids for single-molecule sequencing.

### BACKGROUND OF THE INVENTION

Single molecule sequencing (SMS) platforms, such as nanopore based platforms enable base sequences to be read directly from individual strands of DNA in real-time. Though capable of long read lengths, SMS platforms currently suffer from low throughput compared to competing short-read sequencing platforms. At the same time, many sequencing applications such as oncology and prenatal testing inherently use short nucleic acid fragments such as cell-free DNA (cfDNA) or circulating tumor DNA (ctDNA) present in trace amounts in maternal blood or cancer patient's blood. (*See* Newman, A., et al., (2014) An ultrasensitive method for quantitating circulating tumor DNA with broad patient coverage, Nature Medicine doi:10.1038/nm.3519.) The problem has been addressed in previous publications such as WO 2012/012037A1, WO 2013/040060A2, M. J. FULLWOOD et al.) (GENOME RESEARCH, vol. 19, no. 4, 1 April 2009 (2009-04-01), pages 521-532. and SEAN C. SLEIGHT et al.(ACS SYNTHETIC BIOLOGY, vol. 2, no. 9, 10 (2013-07-10), pages 506-518, "Supplementary Info",) but there is a need for a method of adapting various nucleic acid targets to harnessing the advantages of long read lengths of SMS platforms.

### SUMMARY OF THE INVENTION

In some embodiments, the invention is a method of making a library of concatenated target nucleic acid molecules from a sample, the method comprising: attaching a first adaptor having at least one double-stranded region to each end of a double-stranded target molecule; contacting the sample with an exonuclease to generate partially single-stranded adaptor regions at the ends of the target molecule; joining at least two target molecules by hybridizing the partially single-stranded adaptor regions on each strand of the target molecules to form the double stranded adaptor regions and covalently linking the strands of the target molecules, thereby generating concatenated target molecules; attaching a second adaptor to the concatenated molecules, the adaptor comprising one or more of barcodes, universal amplification priming sites and sequencing priming sites thereby generating a library of concatenated target nucleic acid molecules. The first adaptor may be attached by amplifying the target nucleic acid molecules with primers incorporating the adaptor sequences, or by ligation to the ends of the target nucleic acid molecules. The exonuclease may possess a 5'-3'activity and lacks the 3'-5- activity. The joining of the target molecules may comprise a polymerase fill-in, wherein the polymerase may lack the 3'-5' exonuclease activity. In some embodiments, the joining of the target molecules may comprise a ligation step. In some embodiments, the concatenated products may be purified prior to the step of attaching the second adaptor.

In some embodiments, the method further comprises a step of sequencing the library of concatenated target nucleic acid molecules. The concatenated target nucleic acid molecules may be fractionated by size prior to sequencing. The sequence may be obtained by a method selected from biological nanopore-based method, solid-state nanopore-based method and Single Molecule Real Time (SMRT®)-based method.

In some embodiments, the first adaptor comprises a mixture of adaptors capable of ligation on both ends and adaptors capable of ligation on only one end. The first adaptor may comprise an exonuclease resistant region at least about 15 bases from the 5'-end. In some embodiments, the exonuclease resistant region comprises at least one phosphorothioate nucleotide. In some embodiments the second adaptor comprises a stem-loop structure. In some embodiments the second adaptor consists of at least one double-stranded portion and at least one single-stranded loop that together form a hairpin structure.

In some embodiments, the target molecules are amplified prior to the initial exonuclease treatment. In some embodiments the concatenated molecules are amplified prior to the ligation of the second adaptor.

In some embodiments, the invention is a library of concatenated target nucleic acid molecules created using the method as disclosed above comprising: attaching a first adaptor having at least one double-stranded region to each end of a double-stranded target molecule; contacting the adaptor-containing double-stranded target molecules with an exonuclease to generate partially single-stranded adaptor regions at the ends of the target molecule; joining at least two target molecules by hybridizing the partially single-stranded adaptor regions on each strand of the target molecules to form the double-stranded adaptor regions and covalently linking the strands of the target molecules, thereby generating concatenated target molecules; attaching a second adaptor to the concatenated molecules, the adaptor comprising one or more of barcodes, universal amplification priming sites and sequencing priming sites thereby generating a library of concatenated target nucleic acid molecules.

In some embodiments, the invention is a kit for producing a library of concatenated target nucleic acid molecules according to the method disclosed above, comprising: a first adaptor having at least one double-stranded region, a second adaptor comprising one or more of barcodes, universal amplification priming sites and sequencing priming sites, an exonuclease, a nucleic acid polymerase, and a nucleic acid ligase. The kit may further comprise amplification primers complementary to the first adaptor sequences, a thermostable nucleic acid polymerase and a mixture of at least four deoxynucleoside triphosphates.

In some embodiments, the invention is a method of making a library of concatenated target nucleic acid molecules from a sample, the method comprising: attaching an adaptor molecule to at least one end of a double-stranded target nucleic molecule, wherein an adaptor comprises a rare-cutting restriction endonuclease recognition site to form an adaptor-ligated target molecule; digesting the adaptor-ligated target molecule with the rare-cutting restriction endonuclease to form partially single-stranded termini; joining at least two endonuclease-digested adaptor-ligated target molecules by hybridizing and covalently joining the partially single-stranded termini thereby generating concatenated target molecules. In some embodiments, the adaptor is attached by amplifying the target nucleic acid molecules with primers incorporating the rare-cutting restriction endonuclease recognition site. In some embodiments, the primers further comprise a target-specific sequence and a molecular barcode or a random sequence and a molecular barcode. The adaptor may be attached by ligation to the ends of the target nucleic acid molecules. The rare-cutting restriction endonuclease recognition site may be 10 or more bases long. The rare-cutting restriction endonuclease is a homing restriction endonuclease, e.g., See I or VDE.

In some embodiments, the endonuclease-digested adaptor-ligated target molecules are purified prior to the step of concatenation.

In some embodiments, the adaptor comprises a barcode sequence.

In some embodiments, the method further comprises a step of attaching a second adaptor to at least one end of the concatenated molecules, the adaptor comprising at least one sequencing primer binding site. In some embodiments, the method further comprises a step of sequencing the library of concatenated target nucleic acid molecules. The concatenated target nucleic acid molecules may be fractionated by size prior to sequencing, e.g., by addition of a precipitant.

The sequence is obtained by a method selected from biological nanopore-based method, solid-state nanopore-based method and Single Molecule Real Time (SMRT®)-based method.

In some embodiments, the invention is a method of making concatenated target nucleic acid molecules from a sample, the method comprising: attaching an adaptor molecule to at least one end of a double-stranded target nucleic molecule, wherein an adaptor comprises a rare-cutting restriction endonuclease recognition site to form an adaptor-ligated target molecule; hybridizing a primer to each strand of the adaptor-ligated target molecule wherein the primer comprises a rare-cutting restriction endonuclease recognition site; extending the primer to form from each strand of the adaptor-ligated target molecule, a new molecule containing the rare-cutting restriction endonuclease recognition site on each terminus, digesting the new molecules with the rare-cutting restriction endonuclease to form partially single-stranded termini; joining at least two endonuclease-digested new molecules by hybridizing and covalently joining the partially single-stranded termini thereby generating concatenated target molecules. The primer may comprise a target-specific sequence and a molecular barcode. In some embodiments, the method further comprises a step of amplifying the new molecules. In some embodiments, the method further comprises a step of attaching a second adaptor to at least one end of the concatenated molecules, the adaptor comprising at least one sequencing primer binding site and sequencing the concatenated target nucleic acid molecules.

In some embodiments, the invention is a library of concatenated target nucleic acid molecules created using the method comprising: attaching an adaptor molecule to at least one end of a double-stranded target nucleic molecule, wherein an adaptor comprises a rare-cutting restriction endonuclease recognition site to form an adaptor-ligated target molecule; digesting the adaptor-ligated target molecule with the rare-cutting restriction endonuclease to form partially single-stranded termini; joining at least two endonuclease-digested adaptor-ligated target molecules by hybridizing and covalently joining the partially single-stranded termini thereby generating concatenated target molecules.

In some embodiments, the invention is a kit for producing a library of concatenated target nucleic acid molecules comprising: an adaptor comprising a rare-cutting restriction endonuclease recognition site and a molecular barcode, a second adaptor comprising a universal priming site, a rare-cutting restriction endonuclease and a nucleic acid ligase. The kit may further comprise primers complementary to the universal priming sites, a thermostable nucleic acid polymerase and a mixture of at least four deoxynucleoside triphosphates.

### BRIEF DESCRIPTION OF THE DRAWINGS

**Figures 1(a)****-(c)** illustrate a method of joining short DNA amplicons into long concatemers. **Figure 1(a)** is a diagram of the embodiment of the concatenation method of the invention with adaptors in PCR primers. **Figure 1(b)** is a gel electrophoresis image showing accumulation of concatenates. **Figure 1(c)** is a histogram showing sizes of circular consensus sequence reads of the concatenated sample.

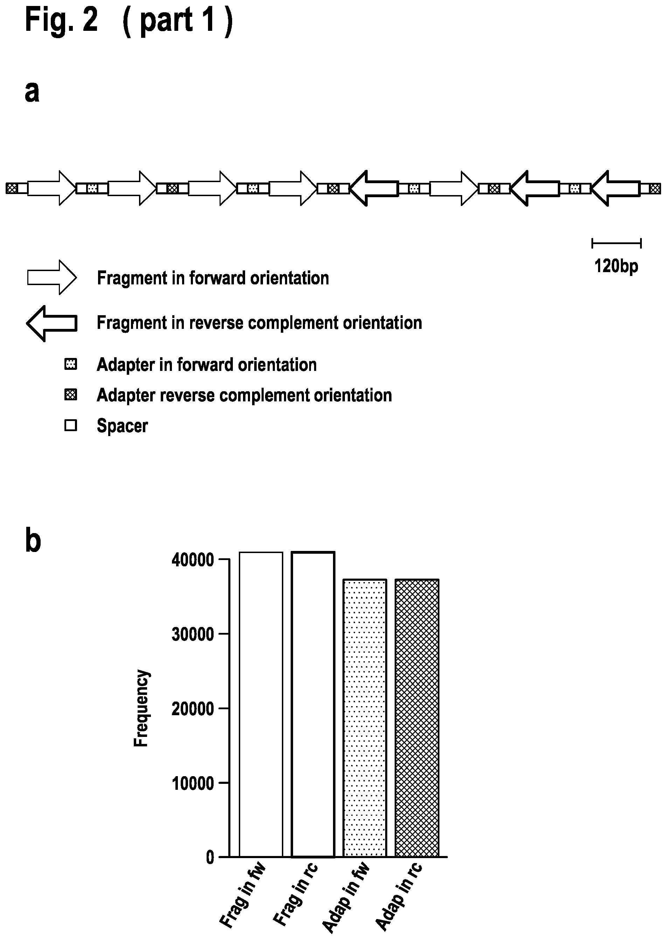

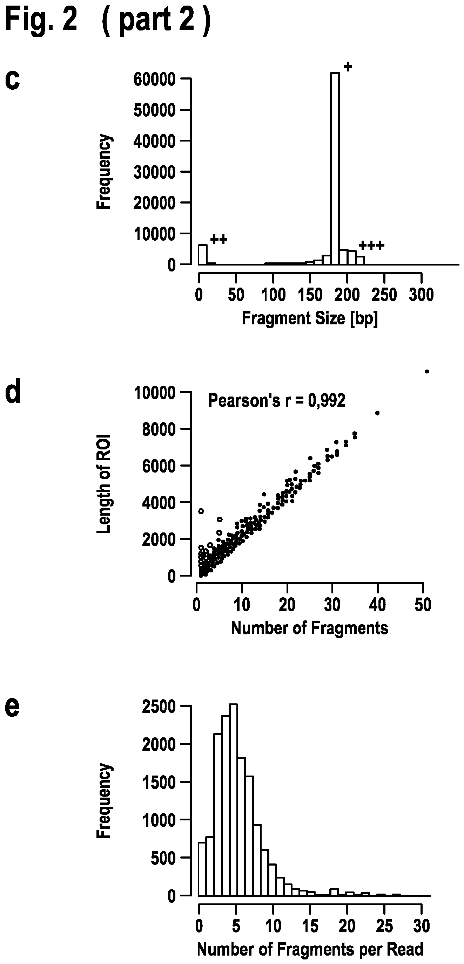

**Figures 2(a)****-(e)** illustrate that the inventive method increases sequencing throughput by more than five-fold. **Figure 2(a)** is a diagram of an exemplary sequence read depicting types and orientation of different sequence features. **Figure 2(b)** is a histogram depicting the number of fragments and adapters in forward and reverse complement orientation identified in all reads. **Figure 2(c)** is a histogram depicting the frequency of fragments in each size bin. **Figure 2(d)** is a scatterplot depicting the relationship between read length and number of fragments identified in that read. **Figure 2(e)** is a histogram depicting the frequency of number of fragments identified per read across all reads.

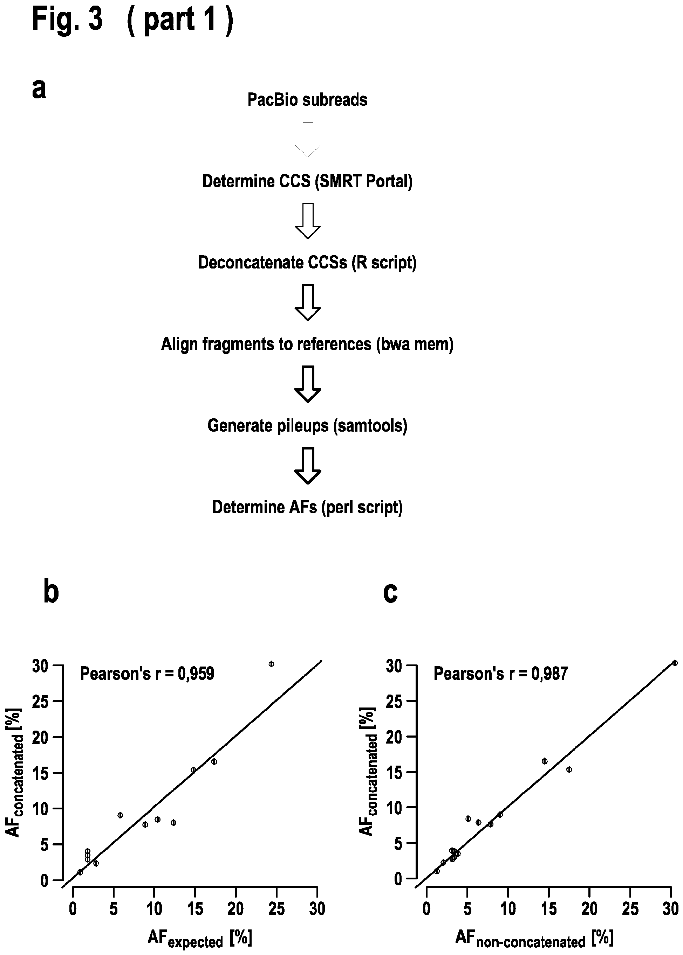

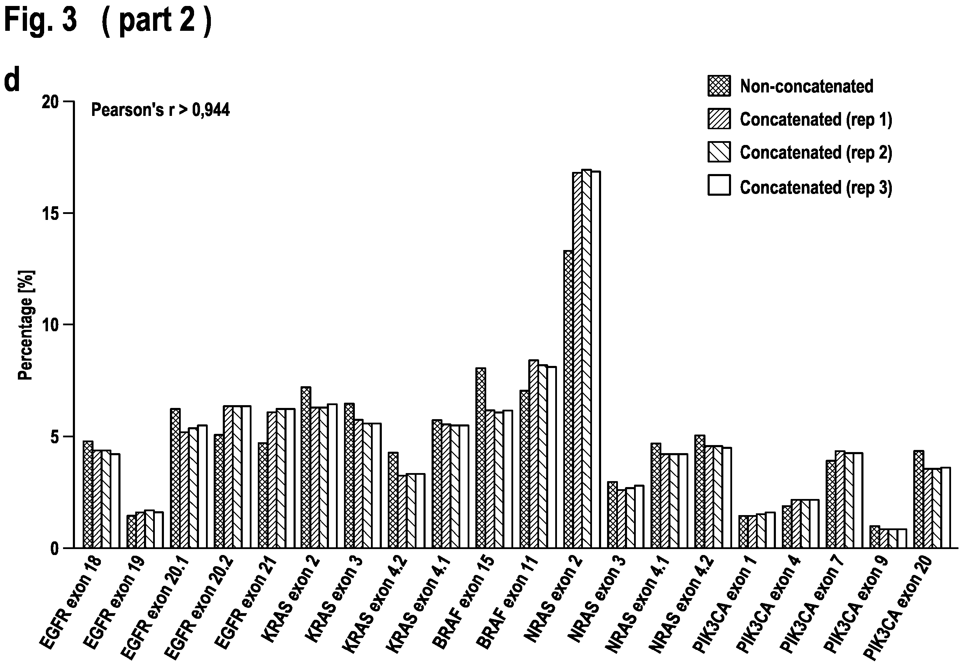

**Figures 3(a)****-(d)** illustrate that the inventive method correctly identifies single-nucleotide variants (SNVs) in an oncology amplicon panel. **Figure 3(a)** is a diagram of an exemplary bioinformatics analysis pipeline used in the invention. **Figure 3(b)** is a scatterplot showing comparison of allele frequencies (AFs) of known single-nucleotide variants in the input DNA) identified in replicates of concatenation samples plotted against the expected frequencies. **Figure3(c)** is a scatterplot showing a comparison of AFs identified in replicates of concatenation samples plotted against frequencies found in the non-concatenation sample. **Figure 3(d)** is a bar plot comparing amplicon coverage in non-concatenated and three replicates of concatenation samples.
**Figures 4(a)****-(c)** illustrate adaptation of inventive method to an alternative target enrichment workflow. **Figure 4(a)** is a diagram of the embodiment of the concatenation method of the invention where target molecules are prepared for adapter ligation by end-repair and A-tailing (ERAT). **Figure 4(b)** is a gel electrophoresis image showing accumulation of concatenates. **Figure 4(c)** is a histogram depicting frequencies of fragment lengths after deconcatenation of concatemer reads.
**Figures 5(a)****-(c)** illustrate how adapters and target sequences assemble during concatenation. **Figure 5(a)** shows one orientation of target-adaptor combination.
**Figure 5(b)** shows another orientation of target-adaptor combination. **Figure 5(c)** shows that 'concatenation units' shown in 5(a) and 5(b) can assemble in two different ways.
**Figures 6(a)****-(c)** illustrate sequencing of concatenated target sequences. **Figure 6(a)** is a gel electrophoresis image showing accumulation of concatenates. **Figure 6(b)** is an electrophoregram of a low-molecular weight DNA ladder. **Figure 6(c)** is an electrophoregram after adaptor ligation and selective amplification of adaptor ligated fragments. **Figure 6(d)** is a scatterplot comparing number of sequenced fragments from LMW-concatemer sequencing run and a run with the adapter-ligated and PCR-amplified LMW.

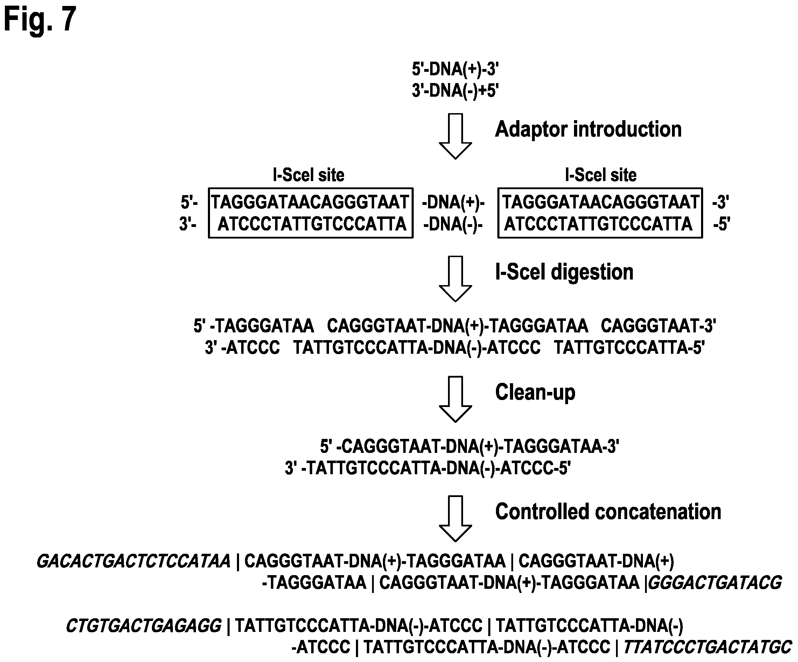

**Figure 7** illustrates the variation of the method with adaptor ligation.
**Figure 8** illustrates the variation of the method with primer extension.
**Figure 9** illustrates the variation of the method with ligation followed by primer extension.
**Figure 10** shows the results of a controlled-size concatenation experiment.

### DETAILED DESCRIPTION OF THE INVENTION

In a first aspect, the present invention provides a method of making a library of concatenated target nucleic acid molecules from a sample, the method comprising:
a. attaching a first adaptor having at least one double-stranded region to each end of a double-stranded target molecule;
b. contacting the sample with an exonuclease to generate partially single-stranded adaptor regions at the ends of the target molecule;
c. joining at least two target molecules by hybridizing the partially single-stranded adaptor regions on each strand of the target molecules to form the double stranded adaptor regions and covalently linking the strands of the target molecules, thereby generating concatenated target molecules; and
d. attaching a second adaptor to the concatenated molecules, the adaptor comprising one or more of barcodes, universal amplification priming sites and sequencing priming sites thereby generating a library of concatenated target nucleic acid molecules.

The first adaptor may be attached by amplifying the target nucleic acid molecules with primers incorporating the adaptor sequence or by ligation to the ends of the target nucleic acid molecules.

The exonuclease in step b may possess a 5'-3'activity and lacks the 3'-5- activity. The joining of the target molecules in step c. comprises a polymerase fill-in. Then, the polymerase may lack the 3'-5' exonuclease activity.

The joining of the target molecules in step c may comprise a ligation step.The concatenated products are purified prior to the step of attaching the second adaptor. The inventive method may further comprise an step of sequencing the library of concatenated target nucleic acid molecules. In this case, the concatenated target nucleic acid molecules may be fractionated by size prior to sequencing. The sequence may be obtained by a method selected from biological nanopore-based method, solid-state nanopore-based method and Single Molecule Real Time (SMRT®)-based method.

The first adaptor may comprise a mixture of adaptors capable of ligation on both ends and adaptors capable of ligation on only one end. The first adaptor may also comprise an exonuclease resistant region at least about 15 bases from the 5'-end, which may comprise at least one phosphorothioate nucleotide. The second adaptor may comprise a stem-loop structure or may consist of at least one double-stranded portion and at least one single-stranded loop that together form a hairpin structure. The method of claim 1, wherein the target molecules may be amplified prior to the exonuclease treatment in step b. The concatenated molecules are amplified prior to the ligation of the second adaptor in step d.

In a second aspect, the present invention provides a library of concatenated target nucleic acid molecules created using the method comprising:
a. attaching a first adaptor having at least one double-stranded region to each end of a double-stranded target molecule;
b. contacting the adaptor-containing double-stranded target molecules with an exonuclease to generate partially single-stranded adaptor regions at the ends of the target molecule;
c. joining at least two target molecules by hybridizing the partially single-stranded adaptor regions on each strand of the target molecules to form the double stranded adaptor regions and covalently linking the strands of the target molecules, thereby generating concatenated target molecules;
d. attaching a second adaptor to the concatenated molecules, the adaptor comprising one or more of barcodes, universal amplification priming sites and sequencing priming sites thereby generating a library of concatenated target nucleic acid molecules.

In a third aspect, the present invention provides kit for producing a library of concatenated target nucleic acid molecules comprising: a first adaptor having at least one double-stranded region, a second adaptor comprising one or more of barcodes, universal amplification priming sites and sequencing priming sites, an exonuclease, a nucleic acid polymerase, and a nucleic acid ligase. The kit may further comprise amplification primers complementary to the first adaptor sequences, a thermostable nucleic acid polymerase and a mixture of at least four deoxynucleoside triphosphates.

In a fourth aspect, the present invention provides method of making a library of concatenated target nucleic acid molecules from a sample, the method comprising:
a. attaching an adaptor molecule to at least one end of a double-stranded target nucleic molecule, wherein an adaptor comprises a rare-cutting restriction endonuclease recognition site to form an adaptor-ligated target molecule;
b. digesting the adaptor-ligated target molecule with the rare-cutting restriction endonuclease to form partially single-stranded termini;
c. joining at least two endonuclease-digested adaptor-ligated target molecules by hybridizing and covalently joining the partially single-stranded termini thereby generating concatenated target molecules.

The adaptor may be attached by amplifying the target nucleic acid molecules with primers incorporating the rare-cutting restriction endonuclease recognition site. The primers may further comprise a target-specific sequence and a molecular barcode. Said rare-cutting restriction endonuclease recognition site may be at least 10 bases long. The rare-cutting restriction endonuclease may be a homing restriction endonuclease or may be selected from See I and VDE. The endonuclease-digested adaptor-ligated target molecules are purified prior to the step of concatenation. The adaptor may also comprise a barcode sequence.

Said method may further comprise a step of attaching a second adaptor to at least one end of the concatenated molecules, the adaptor comprising at least one sequencing primer binding site. Then, a further step of sequencing the library of concatenated target nucleic acid molecules may be executed. If this is the case, the concatenated target nucleic acid molecules may be fractionated by size prior to sequencing by addition of a polymeric precipitant.

In a fifth aspect, the present invention provides a method of making concatenated target nucleic acid molecules from a sample, the method comprising:
a. attaching an adaptor molecule to at least one end of a double-stranded target nucleic molecule, wherein an adaptor comprises a rare-cutting restriction endonuclease recognition site to form an adaptor-ligated target molecule;
b. hybridizing a primer to each strand of the adaptor-ligated target molecule wherein the primer comprises a rare-cutting restriction endonuclease recognition site;
c. extending the primer to form from each strand of the adaptor-ligated target molecule, a new molecule containing the rare-cutting restriction endonuclease recognition site on each terminus;
d. digesting the new molecules with the rare-cutting restriction endonuclease to form partially single-stranded termini;
e. joining at least two endonuclease-digested new molecules by hybridizing and covalently joining the partially single-stranded termini thereby generating concatenated target molecules.

The primer may comprise a target-specific sequence and may further comprise a molecular barcode. The method may further comprise a step of amplifying the new molecules after step c. The method may also comprise a step of attaching a second adaptor to at least one end of the concatenated molecules, the adaptor comprising at least one sequencing primer binding site. If this is the case, a step of sequencing the concatenated target nucleic acid molecules may be added.

In a sixth aspect, the present invention provides a library of concatenated target nucleic acid molecules created using the method comprising:
a. attaching an adaptor molecule to at least one end of a double-stranded target nucleic molecule, wherein an adaptor comprises a rare-cutting restriction endonuclease recognition site to form an adaptor-ligated target molecule;
b. digesting the adaptor-ligated target molecule with the rare-cutting restriction endonuclease to form partially single-stranded termini;
c. joining at least two endonuclease-digested adaptor-ligated target molecules by hybridizing and covalently joining the partially single-stranded termini thereby generating concatenated target molecules.

In a seventh aspect, the present invention provides a kit for producing a library of concatenated target nucleic acid molecules comprising: an adaptor comprising a rare-cutting restriction endonuclease recognition site and a molecular barcode, a second adaptor comprising a universal priming site, a rare-cutting restriction endonuclease and a nucleic acid ligase.

### Definitions

The following definitions aid in understanding of this disclosure.

The term "sample" refers to any composition containing or presumed to contain target nucleic acid. This includes a sample of tissue or fluid isolated from an individual for example, skin, plasma, serum, spinal fluid, lymph fluid, synovial fluid, urine, tears, blood cells, organs and tumors, and also to samples of *in vitro* cultures established from cells taken from an individual patient or from a model organism, including the formalin-fixed paraffin embedded tissues (FFPET) and nucleic acids isolated therefrom. A sample may also include cell-free material, such as cell-free blood fraction that contains cell-free DNA (cfDNA) or circulating tumor DNA (ctDNA).

A term "nucleic acid" refers to polymers of nucleotides (*e.g*., ribonucleotides and deoxyribonucleotides, both natural and non-natural) including DNA, RNA, and their subcategories, such as cDNA, mRNA, *etc.* A nucleic acid may be single-stranded or double-stranded and will generally contain 5'-3' phosphodiester bonds, although in some cases, nucleotide analogs may have other linkages. Nucleic acids may include naturally occurring bases (adenosine, guanosine, cytosine, uracil and thymidine) as well as non-natural bases. Some examples of non-natural bases include those described in, *e.g.,* Seela et al., (1999) Helv. Chim. Acta 82:1640. The non-natural bases may have a particular function, *e.g.,* increasing the stability of the nucleic acid duplex, inhibiting nuclease digestion or blocking primer extension or strand polymerization.

The terms "concatemer" and "concatenate" are used interchangeably and refer to a long continuous nucleic acid molecule that was generated by covalently linking shorter nucleic acids.

The terms "polynucleotide" and "oligonucleotide" are used interchangeably. Polynucleotide is a single-stranded or a double-stranded nucleic acid. Oligonucleotide is a term sometimes used to describe a shorter polynucleotide. An oligonucleotide may be comprised of at least 6 nucleotides or about 15-30 nucleotides. Oligonucleotides are prepared by any suitable method known in the art, for example, by a method involving direct chemical synthesis as described in Narang et al. (1979) Meth. Enzymol. 68:90-99; Brown et al. (1979) Meth. Enzymol. 68:109-151; Beaucage et al. (1981) Tetrahedron Lett. 22:1859-1862; Matteucci et al. (1981) J. Am. Chem. Soc. 103:3185-3191.

The term "primer" refers to a single-stranded oligonucleotide which hybridizes with a sequence in a target nucleic acid ("primer binding site") and is capable of acting as a point of initiation of synthesis along a complementary strand of nucleic acid under conditions suitable for such synthesis. The primer binding site can be unique to each target or can be added to all targets ("universal priming site" or "universal primer binding site").

The terms "adaptor" or "adapter" are used interchangeably and mean a nucleotide sequence that may be added to another sequence so as to import additional properties to that sequence. An adaptor is typically an oligonucleotide that can be single- or double-stranded, or may have both a single-stranded portion and a double-stranded portion. An adaptor may contain sequences such as barcodes and universal primer or probe sites.

The term "ligation" refers to a condensation reaction joining two nucleic acid strands wherein a 5'-phosphate group of one molecule reacts with the 3'-hydroxyl group of another molecule. Ligation is typically an enzymatic reaction catalyzed by a ligase or a topoisomerase. Ligation may join two single strands to create one single-stranded molecule. Ligation may also join two strands each belonging to a double-stranded molecule thus joining two double-stranded molecules. Ligation may also join both strands of a double-stranded molecule to both strands of another double-stranded molecule thus joining two double-stranded molecules. Ligation may also join two ends of a strand within a double-stranded molecule thus repairing a nick in the double-stranded molecule.

The term "barcode" refers to a nucleic acid sequence that can be detected and identified. Barcodes can be incorporated into various nucleic acids. Barcodes are sufficiently long *e.g.,* 2, 5, 10 nucleotides, so that in a sample, the nucleic acids incorporating the barcodes can be distinguished or grouped according to the barcodes.

The terms "multiplex identifier" and "MID" refer to a barcode that identifies a source of a target nucleic acids (*e.g.,* a sample from which the nucleic acid is derived, which is needed when nucleic acids from multiple samples are combined). All or substantially all the target nucleic acids from the same sample will share the same MID. Target nucleic acids from different sources or samples can be mixed and sequenced simultaneously. Using the MIDs the sequence reads can be assigned to individual samples from which the target nucleic acids originated.

The terms "unique molecular identifier" and "UID" refer to a barcode that identifies a nucleic acid to which it is attached. All or substantially all the target nucleic acids from the same sample will have different UIDs. All or substantially all of the progeny (*e.g.,* amplicons) derived from the same original target nucleic acid will share the same UID.

The term "universal primer" and "universal priming binding site" or "universal priming site" refer to a primer and primer binding site present in (typically, *in vitro* added to) different target nucleic acids. For example, the universal priming site may be included in an adaptor ligated to the plurality of target nucleic acids. The universal priming site may also be a part of target-specific (non-universal) primers, for example by being added to the 5'-end of a target-specific primer. The universal primer can bind to and direct primer extension from the universal priming site.

As used herein, the terms "target sequence", "target nucleic acid" or "target" refer to a portion of the nucleic acid sequence in the sample which is to be detected or analyzed. The term target includes all variants of the target sequence, *e.g.,* one or more mutant variants and the wild type variant.

The term "sequencing" refers to any method of determining the sequence of nucleotides in the target nucleic acid.

The cost for sequencing DNA has decreased dramatically over the course of the last ten years at a rate outpacing Moore's law. While we are fast approaching an era in which sequencing an entire human genome costs less than $1,000, it is still not feasible to decipher large numbers of complex genomes, due to reagent costs, informatics infrastructure, time for sample preparation and sequencing. To this end, multiple "target enrichment" methods have been developed in recent years, which selectively enrich for parts of the genome that contain the information of interest. These strategies offer effective ways to lower sequencing cost, increase sequencing depths, shorten sequencing time, and simplify data analysis and they are widely adopted for the detection of genomic variants that can cause human disease. Among the most popular enrichment methods are multiplex PCR, molecular inversion probes, and hybrid capture. These target enrichment approaches typically generate sequencing libraries that contain short DNA molecules (100 - 300 bp) ideally suitable for short-read sequencing platforms such as the array-based cluster generation method with paired-end reads exemplified by the MiSeq and HiSeq systems. (Illumina, San Diego, Cal.) However, alternative sequencing platforms such as single molecule real time (SMRT®) and nanopore-based sequencing are gaining traction.

For example, the single molecule real-time (SMRT®) technology (Pacific BioSciences, Menlo Park, Cal.) uses circular templates containing both strands of the target nucleic acid where the DNA polymerase can generate reads longer than multiple kilobases via multiple passes across both strands. The information from these multiple passes mitigates the relatively high error rate per single pass and is used to generate circular consensus sequence (CCS) reads with high accuracy. Nanopore-based sequencing involves a single DNA polymerase coupled to a membrane-embedded nanopore protein by a short linker. A template and four uniquely tagged nucleotides are added to initiate DNA synthesis. During formation of the ternary complex, a polymerase binds to a complementary tagged nucleotide; the tag specific for that nucleotide is then captured in the pore. Each tag is designed to have a different size, mass, or charge, so that they generate characteristic current blockade signatures, uniquely identifying the added base. *See* Stranges, et al., (2016) Design and characterization of a nanopore-coupled polymerase for single-molecule DNA sequencing by synthesis on an electrode array. PNAS 113(44):E6749.

Long-read technologies, such as SMRT® and nanopore based methods address current limitations of short-read sequencers for *de novo* genome assembly, detection of complex structural variations and characterization of extended repetitive regions in the genome.

However, these long-read technologies currently suffer from low sequencing throughput. On some currently available systems the number of reads generated per run is typically in the tens of thousands. A new generation of instruments is projected to increase the sequencing throughput by approximately seven-fold which will still be at a considerably lower throughput compared to short-read sequencers. This presents a challenge considering sequencing applications that involve short DNA molecules such as cell-free DNA (cfDNA) including circulating tumor DNA (ctDNA) or DNA extracted from formalin fixed paraffin embedded tissues (FFPET). Novel sample preparation strategies in which short DNA fragments are concatenated into long DNA templates could increase the throughput of single molecule sequencers. In addition, such methods would increase the versatility of these platforms to sequence both long and short DNA molecules in a cost-effective way.

In recent years, the synthetic biology community has developed various molecular biology methods to concatenate DNA fragments into genes or gene clusters for the purpose of genome engineering and the production of high added value biomolecules such as pharmaceuticals and biofuels. For example, Gibson Assembly is a method utilizing three enzymes: a 5' exonuclease, a DNA polymerase, and a DNA ligase to covalently link DNA fragments with complementary ends in a simple one-pot isothermal reaction (see U.S. Patent No. 8,968,999). In most Gibson Assembly applications the concatenated fragments are cloned into a vector and subsequently passaged through bacteria for sequence-verification of the desired construct.

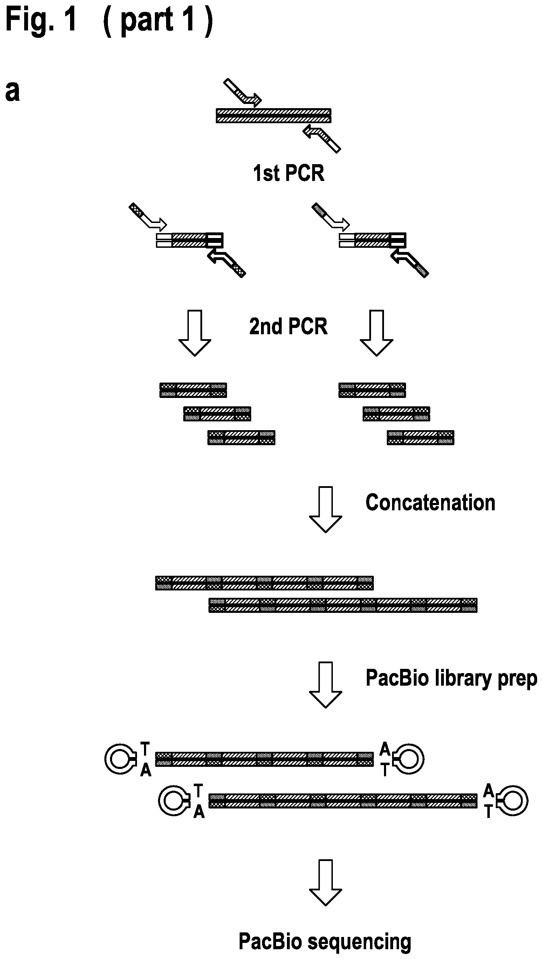

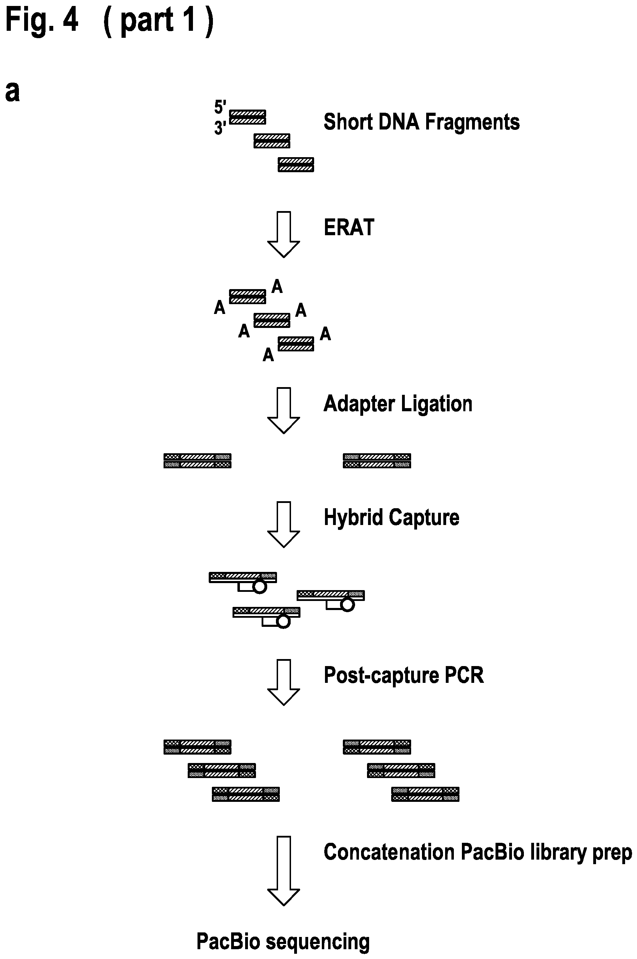

In one embodiment, the invention is a method of generating a library of concatenated nucleic acids for sequencing. Figure 1(a) and Figure 4(a) depict examples of the method according to the invention.

The present invention comprises generating a library of target nucleic acids from a sample for nucleic acid sequencing. Multiple nucleic acids, including all the nucleic acids in a sample may be converted into library molecules using the method and compositions described herein. In some embodiments, the sample is derived from a subject or a patient. In some embodiments the sample may comprise a fragment of a solid tissue or a solid tumor derived from the subject or the patient, *e.g.,* by biopsy. The sample may also comprise body fluids (*e.g.,* urine, sputum, serum, plasma or lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, or fecal samples). The sample may comprise whole blood or blood fractions where normal or tumor cells may be present. In some embodiments, the sample, especially a liquid sample may comprise cell-free material such as cell-free DNA or RNA including cell-free tumor DNA or tumor RNA. In some embodiments, the sample is a cell-free sample, *e.g.,* cell-free blood-derived sample where cell-free tumor DNA or tumor RNA are present. In other embodiments, the sample is a cultured sample, *e.g.,* a culture or culture supernatant containing or suspected to contain nucleic acids derived from the cells in the culture or from an infectious agent present in the culture. In some embodiments, the infectious agent is a bacterium, a protozoan, a virus or a mycoplasma. The sample may also be an environmental sample containing or suspected to contain nucleic acids from organisms.

A target nucleic acid is the nucleic acid of interest that may be present in the sample. In some embodiments, the target nucleic acid is a gene or a gene fragment. In some embodiments, all the genes, gene fragments and intergenic regions (entire genome) constitute target nucleic acids. In some embodiments, only a portion of the genome, *e.g.,* only coding regions of the genome (exome) constitute target nucleic acids. In some embodiments, the target nucleic acid contains a locus of a genetic variant, *e.g.,* a polymorphism, including a single nucleotide polymorphism or variant (SNP of SNV), or a genetic rearrangement resulting *e.g.,* in a gene fusion. In some embodiments, the target nucleic acid comprises a biomarker, *i.e.,* a gene whose variants are associated with a disease or condition. In other embodiments, the target nucleic acid is characteristic of a particular organism and aids in identification of the organism or a characteristic of the pathogenic organism such as drug sensitivity or drug resistance. In yet other embodiments, the target nucleic acid is characteristic of a human subject, *e.g.,* the HLA or KIR sequence defining the subject's unique HLA or KIR genotype.

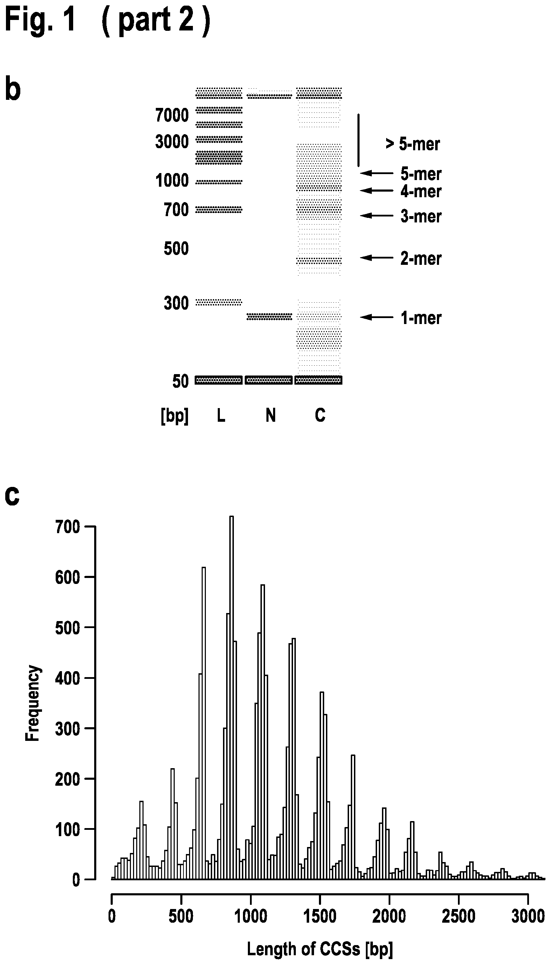

In an embodiment of the invention, one or a plurality of target nucleic acids is converted into the template configuration of the invention. In some embodiments, the target nucleic acid occurs in nature in a single-stranded form (*e.g.,* RNA, including mRNA, microRNA, viral RNA; or single-stranded viral DNA). In other embodiments, the target nucleic acid occurs in nature in a double-stranded form. One of skill in the art would recognize that the method of the invention has multiple embodiments. A single-stranded target nucleic acid can be converted into double-stranded form and then subjected to the steps shown in Figure 1. Longer target nucleic acids may be fragmented by sequence-specific methods (restriction enzymes) or non-specific methods (sonication), although in some applications longer target nucleic acids may be desired to achieve a longer read. In some embodiments, the target nucleic acid is naturally fragmented, *e.g.,* circulating cell-free DNA (cfDNA) or chemically degraded DNA such as the one found in chemically preserved or archived samples.

In the first step, a plurality of double stranded DNA molecules is provided. In some embodiments, the double stranded DNA molecules may be isolated genomic DNA or genomic DNA of reduced complexity (*e.g.,* amplified selected regions of the genome or captured selected regions of the genome such as exome). In some embodiments, the double-stranded DNA is a result of reverse transcription of RNA or other ways of copying a single-stranded nucleic acid into a double-stranded nucleic acid.

In the next step, the double stranded DNA molecules are attached to the first adaptors on each end.

In one embodiment, the adaptors contain a restriction enzyme recognition sequence. It is preferable for the adaptors to contain a rare-cutting recognition sequence that occurs infrequently in the genome. In some embodiments, the recognition sequence is 10 or more bases long. In some embodiments, the recognition sequence is non-palindromic assuring a directional joining of restriction digest fragments. A number of such enzymes are known in the art. *See* Bhagwat, A., (1992) Restriction enzymes: Properties and use, Methods in Enzymology 216:199. In some embodiments, the restriction endonuclease is a homing intron-encoded endonuclease such as See I or VDE. These endonucleases have extremely long recognition sequences (up to 18 base-pairs) that are unlikely to occur more than once in a mammalian genome, and further, these endonucleases generate asymmetric cuts ensuring directional joining of fragments, *see* Jasin, M. (1996) Genetic manipulation of genomes with rare-cutting endonucleases, Trends in Genetics 12:224.

In some embodiments, the template DNA molecule is ligated to an adaptor at each end and has a restriction enzyme recognition sequence on both sides. Following restriction enzyme digestion, multiple template DNA molecules can be joined together. (Figure 1). The adaptors may comprise additional sequences including molecular barcodes and universal primer sites. In some embodiments, adaptors are designed to have the optimal length and GC content. In some embodiments, adaptors of about 10, 15, 20, 30 or 40-bp long are used. In some embodiments, the GC content of the adaptor sequence is about 30%, 40% or 50%.

In some embodiments, the adaptors are attached via extending primers comprising a target-specific portion and an adaptor portion. In some embodiments, the primers are used to perform primer extension or DNA amplification (*e.g.,* PCR) where the primer extension product or the amplicon contains the adaptor sequence. In some embodiments, a single round of primer extension or amplification is performed. In other embodiments, the first round of primer extension or amplification uses primers comprising a target-specific portion and a universal primer binding site. The second round of primer extension or amplification uses universal primers comprising an adaptor sequence.

In some embodiments, the adaptors are ligated to the double stranded target nucleic acid. The adaptors comprise at least one ligatable double-stranded portion. The target nucleic acid comprises ends suitable for ligation or is enzymatically treated to acquire such ends. In some embodiments, the ends of the target nucleic acids are "polished," *i.e.,* extended with a nucleic acid polymerase to ensure double-stranded ends. In some embodiments, the 5'-ends of the target nucleic acids are phosphorylated. In some embodiments, the ligation is a blunt-end ligation. In some embodiments, the ligation is a cohesive end ligation. The 3'-ends of the target nucleic acid are extended with a single nucleotide (*e.g.,* A) and the adaptor is engineered to contain a complementary overhang (*e.g.,* T) at the 3'-ends.

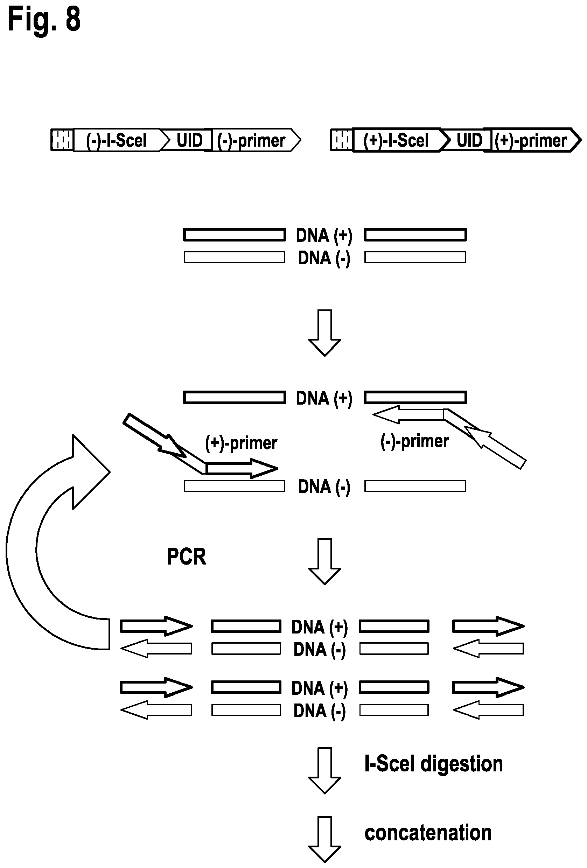

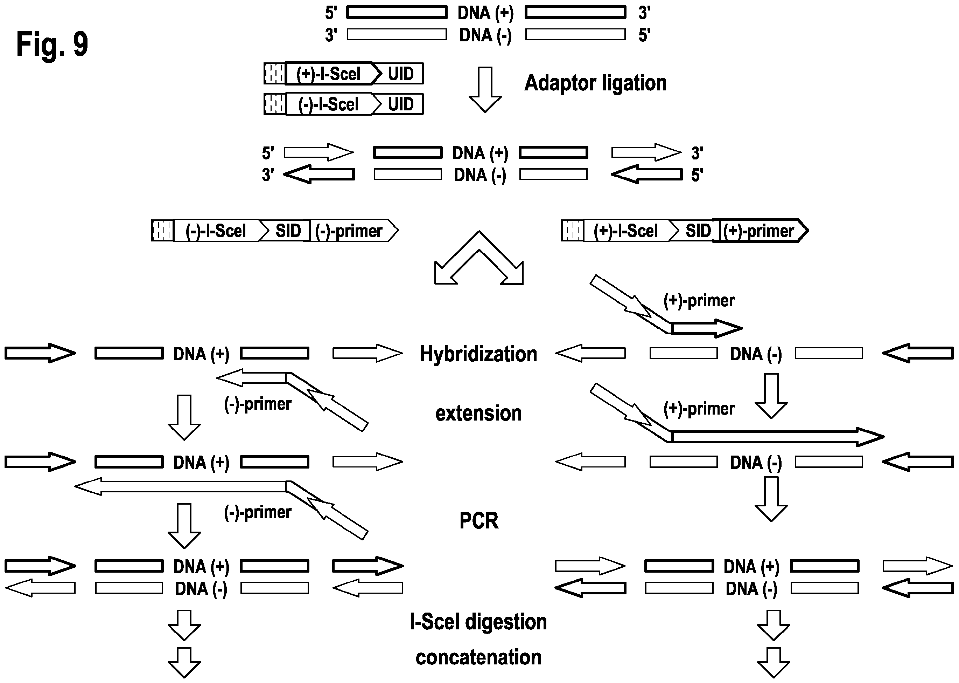

In some embodiments, the restriction enzyme recognition sequences are attached via extending primers comprising a target-specific portion and the restriction enzyme recognition sequence. (Figure 8). In some embodiments, a hybrid approach is used. The double-stranded adaptors are designed to harbor the restriction enzyme recognition sequence in the desired orientation. The adaptors are ligated to both ends of the DNA fragment (Figure 9). Following adaptor ligation, target-specific extension primer is used for each strand ((+) or (-) strand), and harboring both a strand-specific ID (SID) and the restriction enzyme recognition sequence in the desired orientation. The primer is hybridized to one strand or two primers are hybridized separately to each strand of the adaptor-ligated target molecule. The target specific primers and the primer hybridizing to a primer binding site present in the adaptor enable amplification, *e.g.,* by PCR of only desired target molecules from the sample. The amplification products comprise target DNA fragments in desired orientation relative to the restriction enzyme recognition sequence.

The restriction endonuclease is introduced to digest the ends of the adaptor-ligated molecules or products of primer extension. The digestion generates asymmetric molecules with partially single-stranded termini that can be joined only in a certain orientation.

In the next step, the adaptor-ligated target molecules are joined to form concatenates. In some embodiments, at least two, at least three and up to five, ten or more target molecules are joined in a concatenate. This strategy enables the creation of concatenates within which each unit has a desired orientation, facilitating downstream identification and deconvolution of sequence information in each target molecule within the concatenate. For example the use of UIDs allows identifying molecules derived from the same original sequence so that consensus for the molecules could be obtained. Such an approach has broader applications in collating the information from short DNA fragments that typify clinical derived material for the detection of variants associated with cancer.

In some embodiments, the pool of the shorter nucleic acids (being linked together) consists of only one particular species, and therefore the "concatemers" or "concatenates" that are generated contain multiple copies of the same short nucleic acid molecules. In some embodiments, the pool of the shorter nucleic acids (being linked together) consists of multiple different nucleic acid species, and therefore the "concatemers" or "concatenates" that are generated consist of different short nucleic acid molecules (that can, in some cases, occur in multiple copies). In some embodiments, the pool of shorter nucleic acids has been pre-selected by target enrichment approaches (such as, but not limited to, hybrid-capture, multiplex PCR, molecular inversion probe (MIP) technology) before linking them together into concatemers. In some embodiments, the pool of short nucleic acids is not enriched for specific target regions, and represents the entire population of nucleic acid molecules in a sample (for example genomic DNA or cell-free DNA).

In some embodiments, concatenation occurs in a random fashion; new units can be added to both ends of a growing concatemer. Monomers are increasingly depleted and concatemers of higher degrees (such as dimers, trimers, tetramers, etc., collectively termed n-mers) are generated. In an embodiment illustrated in Figure 1(b) the observed lengths of the n-mers are almost exactly of the expected sizes.

In some embodiments, the joining step involves generation and hybridization of complementary or at least partially complementary single stranded ends of the separate molecules. In some embodiments, the complementary or at least partially complementary single stranded ends are generated by contacting adaptor-ligated target nucleic acid molecules with an exonuclease having a 5'-3'-activity. In some embodiments, the exonuclease lacks detectable 3'-5' activity. In some embodiments, the exonuclease is selected from exonuclease T5, exonuclease T7, lambda exonuclease, exonuclease VIII truncated and a mixture thereof.

In some embodiments, the joining step utilizes a DNA polymerase to fill in the gaps in the structures formed by hybridization of complementary or at least partially complementary single stranded ends of the separate molecules. In some embodiments, the DNA polymerase lacks detectable 3'-exonuclease activity. In some embodiments, the DNA polymerase is thermostable. In some embodiments, the DNA polymerase is selected from Taq polymerase, AmpliTaq polymerase and AmpliTaq Gold® polymerase.

In some embodiments, the joining step utilizes a DNA ligase to seal the strands extended by the DNA polymerase. In some embodiments, the DNA ligase is thermostable. In some embodiments, the DNA ligase is selected from T4 DNA ligase, T3 DNA ligase, and a mixture thereof.

In some embodiments, the concatenated target molecules are fractionated by size and the preferred size is selected for further analysis. In some embodiments, fractionation to enrich for larger fragments (larger-order concatenates) is by magnetic bead capture, such as magnetic bead capture in the presence of a crowding agent (Solid Phase Reversible Immobilization (SPRI) technology), preparative gel electrophoresis, including pulse-field gel electrophoresis.

In some embodiments, the invention includes a means of controlling the maximum length of concatemers generated during the concatenation reaction. In some embodiments, the concatenation is limited by using the mixture of adaptors ligatable on both ends and "toxic" adaptors ligatable on only one end. Spiking a suitable (typically much smaller) concentration of "toxic" adaptors will result in capped concatenates that could no longer be extended by further ligation. In some embodiments, the "toxic" adaptor comprises a ligatable double stranded end and a non-ligatable closed-loop hairpin end. In some embodiments, the "toxic" adaptor comprises a ligatable phosphorylated end and a non-ligatable non-phosphorylated end. In some embodiments, the "toxic" adaptor is the second adaptor (described in further detail below) that is used for the sequencing step of the method. In yet another embodiment, the length of concatemers is controlled by introducing an enzyme with alkaline phosphatase activity into the reaction to limit the number of phosphorylated ends of adaptors available for ligation.

In yet other embodiments, the size of concatenates is controlled by size-dependent precipitation. For example, incubation of the ligation reaction in the presence of a polymeric precipitant. In some embodiments, the precipitant is polyethylene glycol (PEG), e.g., PEG 2000, 4000, 6000 or 8000 at a concentration known to sediment DNA exceeding a desired size. In some embodiments, precipitation occurs on solid support and can be controlled or enhanced by additives, e.g., cations such as Mg²⁺. In some embodiments, the addition of MgCl₂ (e.g., at concentrations 5 mM, 10 mM, 20 mM or greater drives sedimentation of concatenates onto the solid support when a concatenate reaches a certain size.

In the next step, the concatenated target molecules are joined with the second adaptor. In some embodiments, the second adaptor enables sequencing of the adaptor-ligated concatenated target molecules. In some embodiments, the second adaptor contains elements required for a particular sequencing platform, *e.g.,* sequencing primer binding sites. In some embodiments, the adaptor is a hairpin adaptor comprising a double-stranded stem portion and a single-stranded loop portion such as described in *e.g.,* U.S. Patent No. 8455193.

In some embodiments, the adaptor comprises one or more barcodes. A barcode can be a multiplex sample ID (MID) used to identify the source of the sample where samples are mixed (multiplexed). The barcode may also serve as a unique molecular ID (UID) used to identify each original molecule and its progeny. The barcode may also be a combination of a UID and an MID. In some embodiments, a single barcode is used as both UID and MID. Another type of barcode is a strand barcode (SID) designed to mark each strand of the target molecule, *e.g.,* a (+) and a (-) strand.

In some embodiments, each barcode comprises a predefined sequence. In other embodiments, the barcode comprises a random sequence. Barcodes can be 1-20 nucleotides long.

In some embodiments, the adaptor further comprises a primer binding site for at least one universal primer. A primer binding site is a sequence complementary to the primer to which primer can bind and facilitate strand elongation.

In some embodiments, the adaptor has more than one *e.g.,* two primer binding sites. In some embodiments, one primer is used for amplification *e.g.,* by PCR (including asymmetric PCR), linear amplification or rolling circle replication (RCA).

The library of adaptor-ligated concatenated target nucleic acids can be sequenced. The template libraries created by the method of the present invention are especially advantageous in single molecule sequencing (SMS) technologies capable of long reads. Examples of such technologies include the Pacific BioSciences platform utilizing the SMRT® technology (Pacific Biosciences, Menlo Park, Cal.) or a platform utilizing nanopore technology such as biological nanopore-based instruments manufactured by Oxford Nanopore Technologies (Oxford, UK) or Roche Genia (Santa Clara, Cal.) or solid state nanopore-based instruments described *e.g.,* in International Application Pub. No. WO2016/142925 and in Stranges, et al., (2016) Design and characterization of a nanopore-coupled polymerase for single-molecule DNA sequencing by synthesis on an electrode array. PNAS 113(44):E6749, and any other presently existing or future single-molecule sequencing technology that is suitable for long reads.

In some embodiments, the sequencing step involves sequence analysis. Sequence analysis may comprise primary and secondary analysis. In some embodiments, the primary analysis comprises analysis performed by the software interfacing with the sequencing instrument and converting signals collected by the instrument (*e.g.,* fluorescent or electrical) into base calls. In some embodiments, the secondary analysis is performed on the primary sequence and comprises sequence aligning. In some embodiments, the secondary analysis further comprises de concatenation.

In some embodiments, deconcatenation includes discreet steps. In some embodiments, the method comprises a step wherein a scanning window slides along each read and makes an approximate matching to the expected adapter sequence. In some embodiments, 1, 2, 3, 4 or more mismatches tolerated including deletions and insertions during matching of the adaptor sequence depending on the length of the adaptor used. In some embodiments the position of adaptors in each read are located by computational methods such as BLAST. These methods further comprise a step of generating a list of adapter and fragment positions in every read. In some embodiments, after deconcatenation the fragments are aligned to the genome or subgenomic fraction such as a list of sequences from the target genomic regions.

In some embodiments, the sample contains target nucleic acids of similar sizes. For example, in some embodiments, the target nucleic acid is a single gene or gene region isolated and amplified from the sample. In other embodiments, the target nucleic acid is a library of sequences of the same length, *e.g.,* cell-free DNA found in human blood including cell-free fetal DNA found in the blood of the mother. Such DNA is on average 150 bp long. In some embodiments, the number or percentage of reads of expected size may be calculated. In other embodiments, the average length of a concatenate can be calculated. *E.g.,* the calculation illustrated in Table 1 demonstrates that on average, each read contained 5.68 fragments.

In some embodiments, the method of the present invention by virtue of concatenation increases the sequencing throughput compared to sequencing a pool of non-concatenated fragments. For example, depending on degree of concatenation, the throughput may be increased 2, 3, 4, 5 or more times.

**Table 1. Overview of PacBio sequencing runs**

| Figure(s) | DNA source | # of total reads | # of fragments | degree of concatenation | # of aligned fragments | on-target rate |
|---|---|---|---|---|---|---|
| 1 and 2 | NRAS (exon 3) | 14,739 | 83,678 | 5.68 | 82,008 | 98.0% |
| 3 | Cancer panel (NC) | 15,143 | 15,143 | 1 | 14,700 | 97.1% |
| 3 | Cancer panel (C-1) | 18,561 | 98,250 | 5.29 | 94,892 | 96.6% |
| 3 | Cancer panel (C-2) | 26,601 | 134,146 | 5.04 | 128,971 | 96.1% |
| 3 | Cancer panel (C-3) | 20,686 | 108,078 | 5.22 | 104,562 | 96.7% |
| 4 | EGFR locus | 52,341 | 231,801 | 4.43 | 224,595 | 97.2% |
| Supp. 1 | LMW DNA ladder | 48,183 | 181,901 | 3.78 | 148,300 | 81.5% |

| | | | | | | |
|---|---|---|---|---|---|---|
| '# of' stands for 'number of'; this excludes all fragments that are only 1 bp long; this is the ratio of # of fragments and # of total reads; this is the fraction of aligned reads of # of total reads; NC: non-concatenated pool; C-1,2,3: concatenated pool, replicates 1,2,3; | | | | | | |

The present invention is a novel method of preparing a sequencing library "ConcatSeq and a related method utilizing rare-cutter restriction enzymes."ConcatSeq". The method is capable of increasing sequencing throughput of single molecule sequencing (SMS) platforms by more than five-fold per run compared to a non-concatenated sample. In some embodiments, the average number of fragments detected across all sequencing reads can be observed as about five. In some embodiments, much longer concatemers, consisting of up to 50 fragments, have been detected. In some embodiments, the potential to increase the sequencing throughput far beyond the five-fold is achieved by applying size selection to the library before sequencing.

In some embodiments, accuracy of the sequence determination depends on the consensus sequence obtained from reading several copies of the target sequence. For example, the accuracy of PacBio's SMRT® technology depends on circular consensus sequence (CCS) reads determined from multiple passes across both strands of the template. Thus, there exists an inherent upper limit to the length of concatemers that yield useful sequencing information. For example, current statistics show that PacBio's accuracy reaches 99% with 5 complete passes and the average length of polymerase reads is between 10 - 15 kb making the ideal length of a concatenated sequencing library between two and four kb. Assuming that short fragments generated by target enrichment workflows are typically around 200 bp, we estimate that our method can be further optimized to increase PacBio sequencing throughput to 10 - 20-fold.

In order to control the maximum length of concatemers generated during the concatenation reaction, we envision (in addition to the strategies listed above for size selection) an approach that uses spike-ins of adapters that will cap a molecule on one or both ends. A non-limiting example of such adaptors is the PacBio-specific hairpin adapters. The toxic adaptor would prevent the concatenate from growing further. The starting concentration of such "toxic" adapters could be used to control the size distribution of the final library.

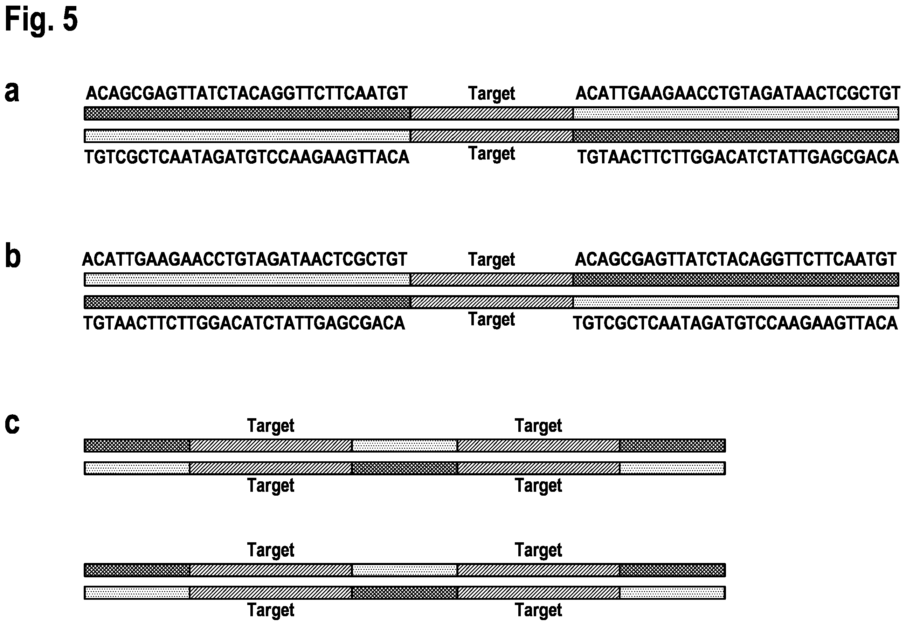

The Examples described herein illustrate validation of the method of the invention by correctly detecting known SNVs in a well-characterized DNA sample. A comparison with known allele frequencies and the representation of molecules in the original pool showed very high concordance with the non-concatenated sample, demonstrating that Gibson Assembly does not significantly increase error rate or sampling bias and corroborating the validity of ConcatSeq (*See* Fig. 3(c) and Fig. 3(d)). The accuracy of the sequence determination using the methods described herein could be further improved by only including 'high-quality' reads, *e.g.* CCS reads with at least 5 passes, and/or by balancing the PCR reactions to ensure equimolar representation of each amplicon. While the examples described herein focused on an oncology target panel with very short fragments (between 80 - 220 bp in length), the experiments using the LMW ladder (Fig. 5 (a)-(c)) demonstrate that ConcatSeq is applicable to concatenating much longer fragments and can therefore be applied in other research areas.

The method of the invention can be readily applied to various target enrichment workflows, as demonstrated by multiplex PCR and workflows where sequencing adapters are incorporated through ligation, such as hybrid capture. Similar solutions can be applied to other assays, such as HEAT-Seq based on molecular inversion probes (Roche Sequencing Solutions, Madison, Wise.). In this case, the only modification to the original protocol is the use of primers that contain ConcatSeq adaptors or adaptors with rare-cutter restriction enzyme sites during the amplification of the circularized target molecule.

Because of the ease with which the method described here can be adapted to different target enrichment schemes, while minimally modifying their original workflow, the instant concatenation methods and their variations provide a powerful and versatile new sample preparation tool for long-read sequencing technologies, including but not limited to PacBio platforms and nanopore-based platforms.

In some embodiments, the invention is a library of concatenated nucleic acid sequences suitable for sequencing. The library comprises concatenated first adaptor-ligated target nucleic acids that are further flanked by the second adaptor. The library is generated by a method comprising the steps of attaching an adaptor molecule to at least one end of a double-stranded target nucleic molecule, wherein an adaptor comprises a rare-cutting restriction endonuclease recognition site to form an adaptor-ligated target molecule; digesting the adaptor-ligated target molecule with the rare-cutting restriction endonuclease to form partially single-stranded termini; joining at least two endonuclease-digested adaptor-ligated target molecules by hybridizing and covalently joining the partially single-stranded termini thereby generating concatenated target molecules.

In some embodiments, the invention is another library of concatenated nucleic acid sequences suitable for sequencing. The library comprises concatenated first adaptor-ligated target nucleic acids that are further flanked by the second adaptor. The library is generated by a method comprising the steps of attaching a first adaptor having at least one double-stranded region to each end of a double-stranded target molecule; contacting the adaptor-containing double-stranded target molecules with an exonuclease to generate partially single-stranded adaptor regions at the ends of the target molecule; joining at least two target molecules by hybridizing the partially single-stranded adaptor regions on each strand of the target molecules to form the double stranded adaptor regions and covalently linking the strands of the target molecules, thereby generating concatenated target molecules; and attaching a second adaptor to the concatenated molecules, the adaptor comprising one or more of barcodes, universal amplification priming sites and sequencing priming sites thereby generating a library of concatenated target nucleic acid molecules.

In some embodiments, the invention is a kit for producing a library of concatenated target nucleic acid molecules comprising: an adaptor comprising a rare-cutting restriction endonuclease recognition site and a molecular barcode, a second adaptor comprising a universal priming site, a rare-cutting restriction endonuclease and a nucleic acid ligase, and optionally, primers complementary to the universal priming sites, a thermostable nucleic acid polymerase and a mixture of at least four deoxynucleoside triphosphates.

In some embodiments, the invention is another kit for producing a library of concatenated target nucleic acid molecules comprising: a first adaptor having at least one double-stranded region, a second adaptor comprising one or more of barcodes, universal amplification priming sites and sequencing priming sites, an exonuclease, a nucleic acid polymerase, and a nucleic acid ligase, and optionally, also amplification primers complementary to the first adaptor sequences, a thermostable nucleic acid polymerase and a mixture of at least four deoxynucleotide triphosphates.

### EXAMPLES

### Example 1 Creating a library of concatenated target molecules

**DNA, oligonucleotides, reagents and kits.** In this example, commercially available genomic DNA from a KRAS-mutant human cell line was purchased from Horizon Discovery (HD701) and Promega (G1471). Low molecular weight DNA ladder was purchased from New England BioLabs (N3233). Oligonucleotides and Nuclease-Free Duplex Buffer were purchased from Integrated DNA Technologies. One oligonucleotide was modified internally by the incorporation of an amino-group in the cytosine. NEBuilder HiFi DNA Assembly Master Mix and Phusion High-Fidelity DNA Polymerase were purchased from New England BioLabs (E2621). Exonuclease III (M0379) and Exonuclease VII (M0206) were purchased from New England BioLabs. AmpliTaq Gold DNA Polymerase with Buffer II and MgCl₂ (N8080241), Nuclease-Free Water (AM9937) and reagents for Qubit dsDNA assays (Q32850 and Q32851) were purchased from Thermo Fisher Scientific. KAPA Hyper Prep Kit (KK8503) and KAPA Pure Beads (KK8002) were purchased from KAPA BioSystems. Agilent DNA 7500 kits (5067-1504) for the Agilent 2100 Bioanalyzer system were purchased from Agilent Technologies.

**PCR amplification and concatenation of target molecules.** For experiments described in Figs. 1-3, target regions of the genome were first amplified using gene-specific primers and 30 ng of HD701 of genomic DNA using AmpliTaq Gold DNA polymerase. This first round of PCR amplified the target regions together with flanking spacers on both ends of each amplicon. For experiments described in Figs. 1(a)-(c) and 2 (a)-(e), the resulting PCR product was then amplified with two primer pairs that prime off the spacer sequences and incorporate complementary ConcatSeq adapters to both ends in two separate PCR reactions. For experiments described in Fig. 3 (a)-(d), the 20 target regions were first amplified in two separate PCR reactions (11 and 9 amplicons, respectively) due to primer incompatibilities. The two PCR products were subsequently amplified in order to incorporate complementary ConcatSeq adapters to their ends. Resulting PCR products were then cleaned using KAPA Pure Beads and quantified using the Qubit dsDNA BR Assay Kit. 200-300 ng of each of the two PCR products were then mixed and the final volume was brought to 40 µl with PCR-grade water. An equal volume (40 µl) of NEBuilder HiFi DNA Assembly Master Mix was added and incubated for 1 h at 50°C. Gibson Assembly was followed by clean-up step using KAPA Pure beads followed by Qubit quantification (typically the concentration was ∼ 10 ng/µl) and size range analysis using Agilent's DNA7500 assay.

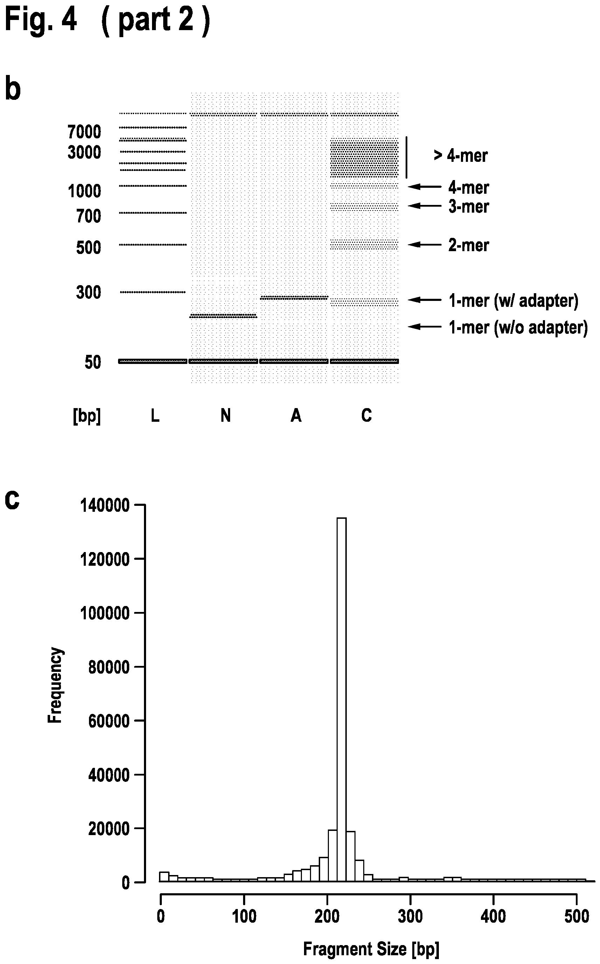

**Ligation of ConcatSeq adapters to target molecules prior to concatenation.** For experiments described in Fig. 4 (a)-(c), two different complementary T-tailed ConcatSeq adapters were generated by annealing PCR primer sequences at 20 µM final concentration. For the experiment described in Figure 4a, four different regions of the EGFR locus were amplified from human genomic DNA (male). The concentration of PCR products was determined using Qubit dsDNA BR Assay and then pooled at equimolar concentration (∼73 nM). For the experiment described in Fig. 4b LMW DNA ladder from NEB was diluted to 10 ng/µl and used as input material. For both, Fig. 4(a) and 4(b), the DNA samples were split into two reactions (with 25 µl comprising ∼ 250 ng total DNA amount each) and subjected to the KAPA Hyper Prep assay: end-repair, A-tailing, and ligation to the two T-tailed ConcatSeq adapters. The resulting adapter-ligated fragment pools were then PCR-amplified to enrich for the fragments that had successfully ligated adapters on both ends. DNA concentrations were quantified using the Qubit dsDNA BR Assay Kit. 200-300 ng of each of the two PCR products were then mixed and filled up to 40 µl with PCR-grade water. An equal volume of NEBuilder HiFi DNA Assembly Master Mix was added and incubated for 30, 60, 100, and 120 min at 50°C. Gibson Assembly was followed by clean-up step using KAPA Pure beads (0.8x ratio), followed by Qubit quantification and size range analysis of size range using Agilent's DNA7500 assay.

A double-stranded adaptor, harboring both a UID and the restriction site of See I in the desired orientation is ligated to both ends of the DNA fragment (Figure 1). The ligation products are digested by See I and joined by DNA ligase.

**PacBio library preparation.** Approximately 100 ng of the concatenated pool was used to prepare PacBio sequencing libraries using the KAPA Hyper Prep Kit. A suitable T-tailed hairpin adapter was first created by self-annealing of an adaptor oligonucleotide (20 µM) using Duplex Buffer and heating for 5 min to 80°C followed by a slow ramp-down (0.2°C per second) to 25°C. Double-stranded DNA concatemers were then subjected to end-repair and A-tailing, and ligated to the hairpin adapters (at roughly a 250:1 ratio of adapters to concatemers) for 30 min at 20°C. Unreacted T-tailed hairpin adapters and concatenated DNA molecules were removed by adding exonuclease III and exonuclease VII (1 µl of each) to the sample and incubating for 30 min at 37°C. The resulting library molecules were cleaned-up with KAPA Pure Beads at 0.8x ratio, and then quantified using Qubit dsDNA HS Assay. On average the final concentration of the sequencing libraries was between 0.5 and 2 ng/µl.

### Example 2. Sequencing the library of concatenated target molecules

**PacBio sequencing.** Binding Calculator (version 2.3.1) was used to prepare the library for PacBio sequencing using the MagBead one-cell per well (OCPW) protocol, and binding kit P6v2 was used with an on-plate concentration of 0.05 nM. Primer conditioning and annealing, as well as binding of the polymerase to the templates, and complex binding to the magnetic beads was done exactly as indicated by the binding calculator protocol. Templates complexes were incubated with MagBeads for 2 hours at 4°C prior to loading a SMRT cell. Four-hour movies were recorded and primary sequence analysis was performed on the PacBio RSII instrument.

### Example 3. Alternative method of preparing a library of concatenated target nucleic acids

**Alternative method of attaching adaptors.** Figure 4(a) depicts an adaptation for SeqCap method (Roche Sequencing Solutions, Madison, Wise.), where there are only two changes in the workflow. First, the Y-adapters, which are ligated to DNA fragments at the beginning of the protocol, are replaced with ConcatSeq adapters (Fig. 4(a), Adapter Ligation step). Second, a new step is introduced in which the captured and PCR-amplified target molecules are incubated with the enzyme master mix for 1 hour for concatenation to take place.

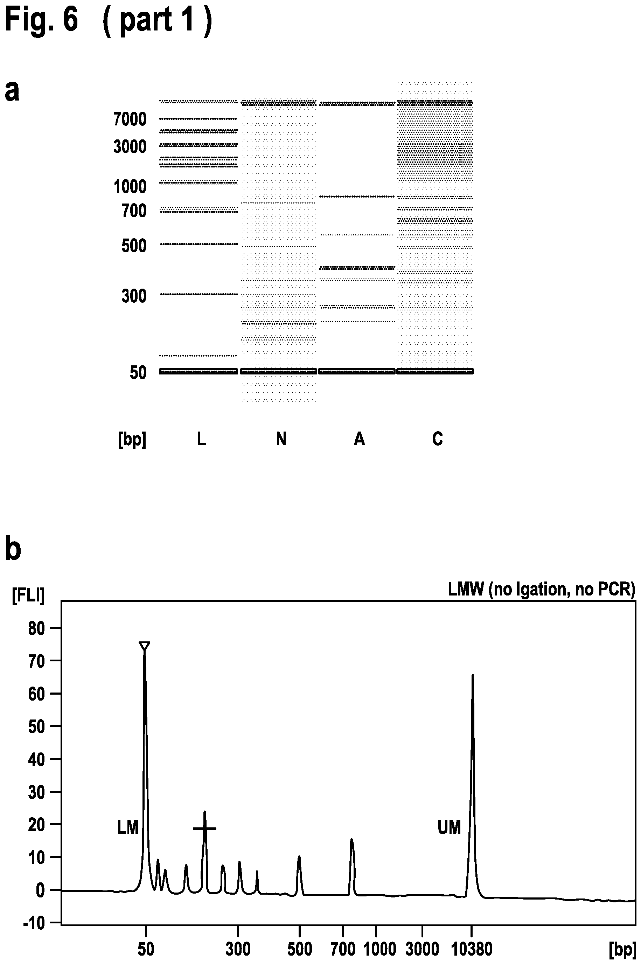

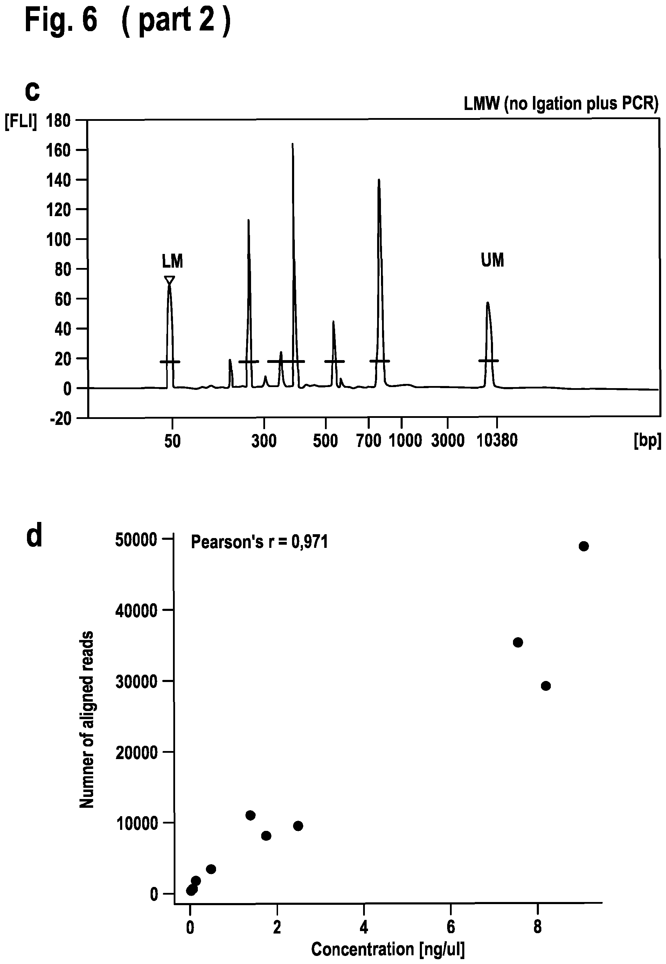

To test whether ConcatSeq works in a situation where ConcatSeq adapters are ligated to DNA fragments, rather than incorporated by PCR amplification, we first generated a pool consisting of four PCR products from the human EGFR locus. The amplicons all had a size of 220 bp and were amplified using male human genomic DNA (G1471, Promega) as a template (Fig. 4(b)). The pooled DNA was split into two aliquots, and two types of overlapping adapters were attached via A-tailed ligation. We performed a PCR step for enrichment prior to concatenation as described before in Fig. 1(a). Note, that this PCR reaction mimics the PCR step in the current workflow in which the target enriched library is amplified pre-sequencing. Average numbers of fragments per read were slightly reduced compared to previous runs. However, on-target rate was excellent, confirming that the ligation-based approach is valid. The large majority of deconcatenated fragments had the expected size of 220 bp (Fig. 4(c)). For a second test, we used a low molecular weight DNA ladder (LMW) containing 11 double-stranded DNAs of varying lengths as a starting material for adapter ligation. In this concatenation experiment, the average number of fragments per read was only 3.8-fold (Table 1), which is expected due to the presence of much larger molecules (up to 766 bp) in the mix. We noticed that representation of the LMW fragments was strongly influenced by adapter ligation and/or subsequent PCR amplification (Fig. 6(a)-(d)). A high correlation (Pearson's r = 0.971) was found between the frequencies of the aligned LMW fragments and fragment concentrations after adapter ligation (Fig. 5(d)), confirming that our method subsamples the molecules with low bias during assembly.

### Example 4. Sequencing data analysis

**Secondary and Tertiary data analysis.** Reads of inserts were determined using the default settings on the SMRT Portal: only reads with more than one full pass and a minimum predicted accuracy of 90% were included for CCS reads generation. The circular consensus sequence reads were deconcatenated using an adapter scanning approach, which we implemented in R. Briefly, a window of 30 bp (which corresponds to the length of the ConcatSeq adapter) slides along each read and performs approximate matching to the ConcatSeq adapter sequence (in forward and reverse complement orientation) using the *agrep* function and allowing for up to 4 mismatches, insertions, and/or deletions. Adapters identified this way are removed from the reads leaving deconcatenated fragments behind. New fastq-files are created which list all adapters and fragments identified by this method. Before alignment of the fragments to the references, all fragments of length 1 bp were removed. Spacer sequences (introduced during the first PCR amplification in experiments described in Figs. 1(a)-(c)-3(a)-(d)) remained part of each fragment after deconcatenation and were not specifically removed before alignment using bwa mem. Spacer sequences flanking each fragment were soft-clipped during alignment. Only alignments that had a samflag of either 0 or 16, indicating a correct alignment in forward or reverse complement orientation, respectively, were kept for further analysis. For Fig. 3 (a)-(d), we generated pileups of the aligned fragments using the *mpileup* function in samtools. We used a Perl script to transform the pileups into contingency tables reporting the frequency of each bases called at every position. Allele frequencies at the relevant positions (*i.e.* the known single nucleotide variants in HD701) were extracted from these tables and plotted as the fraction of total number of reads aligned at that position.

### Example 5. Evaluation of the method of the invention

**ConcatSeq sequencing evaluation.** To confirm that our concatenation approach was successful, we (randomly) chose a read consisting of 1719 bp from ZMW 93 for detailed inspection. Based on its length we suspected that it is an 8-mer. Three recurring features were identified in this read: the 30 bp ConcatSeq adapters, target sequence, and spacers (Fig. 2(a)). (For simplicity we will refer to the target plus flanking spacer sequences as 'targets' or 'fragments' from here on.) As expected from the design used in our approach, adapters switch between forward and reverse complement orientation along the read. The orientation of the targets is random, but both orientations are present at roughly the same frequency (*i.e.* five in forward and three in reverse complement).

To extend this type of analysis to all 14,739 sequencing reads, we implemented a bioinformatics method to automate deconcatenation. This method is based on an algorithm where a scanning window slides along each read and makes an approximate matching (with up to 4 mismatches tolerated including deletions and insertions) to the expected adapter sequence, and generates lists of adapter and fragment positions in every read. As expected, the number of all fragments in forward and reverse complement orientation was almost exactly equal (Fig. 2(b)). The same was true for the adapters in both orientations. We also observed a smaller number of adapters compared to fragments which we hypothesized was due to the adapters being located at the ends of the concatemers sometimes being truncated and therefore not identified by our adapter scanning approach. Further inspection of the ends of the reads confirmed this hypothesis.

In sum, 89,496 fragments and 75,312 adapters were identified in the 14,739 reads. The vast majority of the targets (n=62,093, 74.2%) had exactly the expected size of 187 bp or was very close (181-190 bp) to the expected size (Fig. 2(c)). Notably, there was a second population of fragments that consisted of only one base (n=5818, 6.5%). All of these fragments were located at the beginning or the end of the reads and the majority of these were either an adenine or a thymidine (85%). These single base fragments are most likely remnants of the hairpin adapters attached to the n-mers via A-tailed ligation during library preparation (Fig. 1(a)). A third population (n= 12,783, 15.3%) consisted of fragments that were slightly longer than the expected size (> 190 bp). Again, the majority of these fragments was located at the ends of the reads and contained the target along with truncated adapter sequences.

We excluded the 5818 single nucleotide fragments from further analysis, leaving 83,678 fragments after deconcatenation (Table 1). On average each read contained 5.68 fragments, indicating that our approach increased the sequencing throughput by at least five-fold compared to sequencing a pool of non-concatenated fragments. Alignment of the targets to the reference sequence showed a superb on-target rate (98.0%), suggesting that concatenation did not interfere with the fidelity of the target sequences. This further corroborates the validity of ConcatSeq.

Because the fragments that were concatenated in this experiment were all of the same size (Fig. 1b, lane [N]), a linear relationship between the length of the read and the number of fragments in that read is expected. This linear relationship was observed for the large majority of reads (Fig. 2(d)). In the remaining 22 reads a few adapter sequences were failed to be identified by our algorithm because they had more than 4 mismatches with the reference sequence. Strikingly, while the majority of reads (70.5%) contained between three and seven fragments (Fig. 2(e)) and were between 600 and 1500 bp in length, we found a wide spread of read lengths, with the longest being more than 10kb in size and containing more than 50 fragments (Fig. 2(d)). This suggests that ConcatSeq has the potential to further increase the sequencing throughput by size selecting for longer concatemers prior to sequencing. **Validation of ConcatSeq by detecting single-nucleotide variants (SNVs) in an oncology amplicon panel.** We next examined whether ConcatSeq can be used to correctly identify known SNVs and their allele frequencies in a biological sample. To this end, we amplified a set of oncology targets by PCR using a well-characterized DNA reference (HD701, Horizon Discovery) as a template. HD701 is a commercially available engineered isogenic cell line in which precise allelic frequencies for major oncology targets have been determined by digital PCR. Allele frequencies (AFs) of the verified variants in this DNA sample are between 1% and 24.5%, allowing the assessment of accuracy and sensitivity of our assay. Twenty amplicons spanning 5 genes (EGFR, KRAS, NRAS, BRAF, and PIK3CA) were generated in two separate multiplex PCRs (containing 11 and 9 targets, respectively), and then flanked by complementary ConcatSeq adapters (Fig. 1(a)). Equimolar amounts of these two amplicon pools were mixed and concatenated in three independent reactions, followed by PacBio sequencing, serving as triplicate samples to assess reproducibility of our assay. As before, on average more than 5 fragments were observed per read in these samples (Table 1). We also sequenced the non-concatenated amplicon pool as a control. A bioinformatics pipeline was then established (Fig. 3(a)) that aligns the deconcatenated and non-concatenated fragments to the 20 reference sequences, generates pileups of each alignment, and subsequently extracts AFs of the known variants in HD701 cell line DNA. The on-target rates in all three concatenated samples and the non-concatenated control were again very high (> 96.1%). Allele frequencies identified with ConcatSeq were highly correlated (Pearson's r = 0.959) between the three replicates of concatenated samples and the expected frequencies (Fig. 3(b)), indicating ConcatSeq's ability to retrieve this information with great accuracy and sensitivity. A comparison of AFs in the concatenated and the non-concatenated control showed even higher concordance (Pearson's r = 0.987), indicating that deviations from the expected frequencies were likely introduced during amplicon generation and not during concatenation or PacBio sequencing. To ensure that our approach does not introduce significant bias into the frequency of amplicons represented in the pool before concatenation, we compared percent coverage of each of the 20 amplicons in the three concatenated samples and the non-concatenated sample (Fig. 3(d)). A very high correlation was found (Pearson's r > 0.944) between these groups, indicating that ConcatSeq subsamples the amplicons from the original pool with low bias.

### Example 6. Creating a library of concatenated target molecules via adaptor ligation

A double-stranded adaptor, harboring both a UID and the restriction site of See I in the desired orientation is ligated to both ends of the DNA fragment (Figure 1). The ligation products are digested by Sce I and joined by DNA ligase.

### Example 7. Creating a library of concatenated target molecules via primer extension

The forward and reverse primers are designed to harbor the restriction site of Sce I in the desired orientation as well as a UID (Figure 2). These primers are used to select regions of interest within a sample via PCR. Following PCR amplification, amplification products are digested by Sce I and joined by DNA ligase.

### Example 8. Creating a library of concatenated molecules via ligation and primer extension

A double-stranded adaptor, harboring both a UID and the restriction site of Sce I in the desired orientation is ligated to both ends of the DNA fragment (Figure 3). An extension primer designed against the (+) or (-) strand, and harboring both a strand-specific ID (SID) and the Sce I restriction site in the desired orientation is hybridized separately to each strand of the target molecule. Following PCR, adapted fragment molecules of a desired insert orientation are generated. Alternatively, extension primers for both strands are simultaneously hybridized to the target molecule. Following PCR, adapted fragment molecules with a random insert orientation are generated. Purified PCR products from both reactions can then be processed as described above.

### Example 9. Creating concatenates of desired size ranges

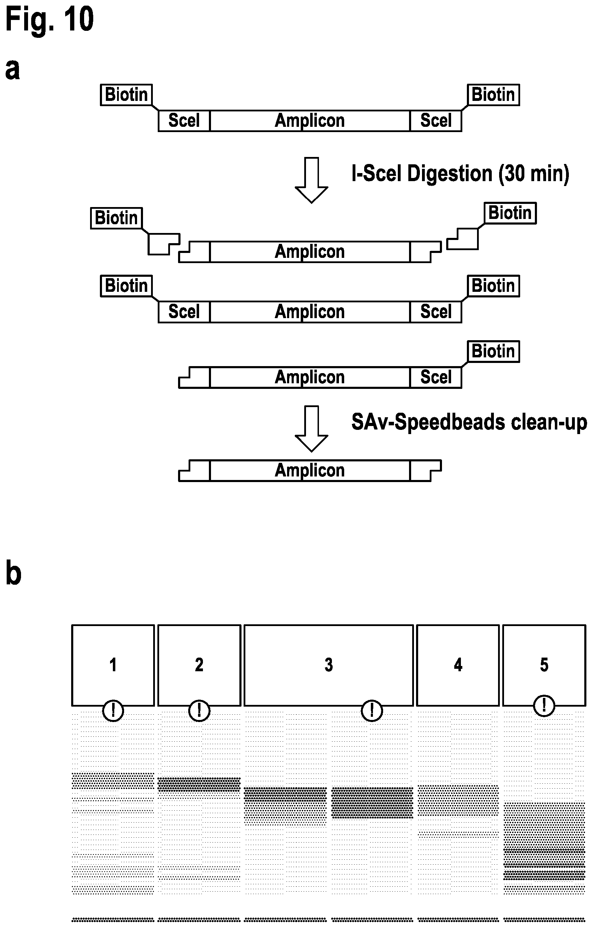

Target DNA fragments are PCR amplified with biotinylated primers to create biotinylated amplicons. These are subjected to digestion with a restriction enzyme SceI to expose non-palindromic overhangs for concatenation. All biotinylated species are removed by incubation with a Streptavidin bound solid support to leave only the fully digested product. (Figure 10, A) Standard ligation reactions using T4 DNA ligase are performed in the presence of carboxylated SeraMag Speedbeads (GE Healthcare Bio-Sciences, Pittsburgh, Penn.) and increasing amounts of PEG-8000. After 30 min, the beads are magnetized, the supernatant is removed and the beads are washed with 70% ethanol. Concatemers are then eluted in TE buffer. Results are shown on Figure 10, B: lanes 1-5 show electrophoresis of ligaiton mixtures with increasing concentrations of PEG 8000 (6%-14% w/v). Lane 6 is no precipitation control.

## Claims

1. A method of making a library of concatenated target nucleic acid molecules from a sample, the method comprising:
a. attaching a first adaptor having at least one double-stranded region to each end of a double-stranded target molecule;
b. contacting the sample with an exonuclease to generate partially single-stranded adaptor regions at the ends of the target molecule;
c. joining at least two target molecules by hybridizing the partially single-stranded adaptor regions on each strand of the target molecules to form the double stranded adaptor regions and covalently linking the strands of the target molecules, thereby generating concatenated target molecules;
d. attaching a second adaptor to the concatenated molecules, the adaptor comprising one or more of barcodes, universal amplification priming sites and sequencing priming sites thereby generating a library of concatenated target nucleic acid molecules.

2. The method of claim 1, wherein the first adaptor is attached by amplifying the target nucleic acid molecules with primers incorporating the adaptor sequences, or the first adaptor is attached by ligation to the ends of the target nucleic acid molecules.

3. The method of claim 1, wherein the first adaptor comprises a mixture of adaptors capable of ligation on both ends and adaptors capable of ligation on only one end.

4. The method of claim 1, wherein the first adaptor comprises an exonuclease resistant region at least about 15 bases from the 5'-end.

5. A library of concatenated target nucleic acid molecules created using the method according to claims 1-4, said method comprising:
a. attaching a first adaptor having at least one double-stranded region to each end of a double-stranded target molecule;
b. contacting the adaptor-containing double-stranded target molecules with an exonuclease to generate partially single-stranded adaptor regions at the ends of the target molecule;
c. joining at least two target molecules by hybridizing the partially single-stranded adaptor regions on each strand of the target molecules to form the double stranded adaptor regions and covalently linking the strands of the target molecules, thereby generating concatenated target molecules;
d. attaching a second adaptor to the concatenated molecules, the adaptor comprising one or more of barcodes, universal amplification priming sites and sequencing priming sites thereby generating a library of concatenated target nucleic acid molecules.

6. Use of a kit for producing a library of concatenated target nucleic acid molecules for a method according to claims 1-4, said kit comprising: a first adaptor having at least one double-stranded region, a second adaptor comprising one or more of barcodes, universal amplification priming sites and sequencing priming sites, an exonuclease, a nucleic acid polymerase, and a nucleic acid ligase, and optionally further comprising amplification primers complementary to the first adaptor sequences, a thermostable nucleic acid polymerase and a mixture of at least four deoxynucleoside triphosphates.

## Patentansprüche

1. Verfahren zum Erstellen einer Bibliothek von verketteten Zielnukleinsäuremolekülen aus einer Probe, wobei das Verfahren Folgendes umfasst:
a. Binden eines ersten Adaptors mit mindestens einer doppelsträngigen Region an jedes Ende eines doppelsträngigen Zielmoleküls;
b. Inkontaktbringen der Probe mit einer Exonuklease unter Erzeugung teilweise einzelsträngiger Adaptorregionen an den Enden des Zielmoleküls;
c. Verbinden von mindestens zwei Zielmolekülen durch Hybridisieren der teilweise einzelsträngigen Adaptorregionen an jedem Strang der Zielmoleküle unter Bildung der doppelsträngigen Adaptorregionen und kovalentes Verknüpfen der Stränge der Zielmoleküle, wodurch verkettete Zielmoleküle erzeugt werden;
d. Binden eines zweiten Adaptors an die verketteten Moleküle, wobei der Adaptor eines oder mehrere von Barcodes, universellen Amplifikations-Priming-Stellen und Sequenzierungs-Priming-Stellen umfasst, wodurch eine Bibliothek von verketteten Zielnukleinsäuremolekülen erzeugt wird.

2. Verfahren nach Anspruch 1, wobei der erste Adaptor durch Amplifizieren der Zielnukleinsäuremoleküle mit Primern, die die Adaptorsequenzen aufnehmen, gebunden wird oder der erste Adaptor durch Ligation an die Enden der Zielnukleinsäuremoleküle gebunden wird.

3. Verfahren nach Anspruch 1, wobei der erste Adaptor ein Gemisch aus Adaptoren, die an beide Enden ligieren können, und Adaptoren, die nur an ein Ende ligieren können, umfasst.

4. Verfahren nach Anspruch 1, wobei der erste Adaptor eine Exonuklease-resistente Region umfasst, die mindestens etwa 15 Basen vom 5'-Ende entfernt ist.

5. Bibliothek von verketteten Zielnukleinsäuremolekülen, die unter Anwendung des Verfahrens nach den Ansprüchen 1-4 geschaffen wurde, wobei das Verfahren Folgendes umfasst:
a. Binden eines ersten Adaptors mit mindestens einer doppelsträngigen Region an jedes Ende eines doppelsträngigen Zielmoleküls;
b. Inkontaktbringen der Adaptor-enthaltenden doppelsträngigen Zielmoleküle mit einer Exonuklease zur Erzeugung teilweise einzelsträngiger Adaptorregionen an den Enden des Zielmoleküls;
c. Verbinden von mindestens zwei Zielmolekülen durch Hybridisieren der teilweise einzelsträngigen Adaptorregionen an jedem Strang der Zielmoleküle unter Bildung der doppelsträngigen Adaptorregionen und kovalentes Verknüpfen der Stränge der Zielmoleküle, wodurch verkettete Zielmoleküle erzeugt werden;
d. Binden eines zweiten Adaptors an die verketteten Moleküle, wobei der Adaptor eines oder mehrere von Barcodes, universellen Amplifikations-Priming-Stellen und Sequenzierungs-Priming-Stellen umfasst, wodurch eine Bibliothek von verketteten Zielnukleinsäuremolekülen erzeugt wird.

6. Verwendung eines Kits zum Produzieren einer Bibliothek von verketteten Zielnukleinsäuremolekülen für ein Verfahren nach den Ansprüchen 1-4, wobei der Kit Folgendes umfasst: einen ersten Adaptor mit mindestens einer doppelsträngigen Region, einen zweiten Adaptor, umfassend eines oder mehrere von Barcodes, universellen Amplifikations-Priming-Stellen und Sequenzierungs-Priming-Stellen, eine Exonuklease, eine Nukleinsäurepolymerase und eine Nukleinsäureligase und gegebenenfalls ferner umfassend Amplifikations-Primer, die komplementär zu den ersten Adaptorsequenzen sind, eine thermostabile Nukleinsäurepolymerase und ein Gemisch aus mindestens vier Desoxynukleosidtriphosphaten.

## Revendications

1. Procédé de fabrication d'une bibliothèque de molécules d'acides nucléiques cibles concaténées à partir d'un échantillon, le procédé comprenant :
a. la fixation d'un premier adaptateur ayant au moins une région bicaténaire à chaque extrémité d'une molécule cible bicaténaire ;
b. la mise en contact de l'échantillon avec une exonucléase pour générer des régions d'adaptateur partiellement monocaténaires aux extrémités de la molécule cible ;
c. la jonction d'au moins deux molécules cibles par hybridation des régions d'adaptateur partiellement monocaténaires sur chaque brin des molécules cibles pour former les régions d'adaptateur bicaténaires et la liaison de manière covalente des brins des molécules cibles, générant ainsi des molécules cibles concaténées ;
d. la fixation d'un second adaptateur aux molécules concaténées, l'adaptateur comprenant un ou plusieurs parmi des codes-barres, des sites d'amorçage d'amplification et des sites d'amorçage de séquençage universels générant ainsi une bibliothèque de molécules d'acides nucléiques cibles concaténées.

2. Procédé selon la revendication 1, dans lequel le premier adaptateur est fixé par amplification des molécules d'acides nucléiques cibles avec des amorces incorporant les séquences d'adaptateur, ou le premier adaptateur est fixé par ligature aux extrémités des molécules d'acides nucléiques cibles.

3. Procédé selon la revendication 1, dans lequel le premier adaptateur comprend un mélange d'adaptateurs capables de ligature sur les deux extrémités et d'adaptateurs capables de ligature sur une seule extrémité.

4. Procédé selon la revendication 1, dans lequel le premier adaptateur comprend une région résistant à une exonucléase au moins à environ 15 bases de l'extrémité 5'.

5. Bibliothèque de molécules d'acides nucléiques cibles concaténées créées en utilisant le procédé selon les revendications 1 à 4, ledit procédé comprenant :
a. la fixation d'un premier adaptateur ayant au moins une région bicaténaire à chaque extrémité d'une molécule cible bicaténaire ;
b. la mise en contact des molécules cibles bicaténaires contenant l'adaptateur avec une exonucléase pour générer des régions d'adaptateur partiellement monocaténaires aux extrémités de la molécule cible ;
c. la jonction d'au moins deux molécules cibles par hybridation des régions d'adaptateur partiellement monocaténaires sur chaque brin des molécules cibles pour former les régions d'adaptateur bicaténaires et la liaison de manière covalente des brins des molécules cibles, générant ainsi des molécules cibles concaténées ;
d. la fixation d'un second adaptateur aux molécules concaténées, l'adaptateur comprenant un ou plusieurs parmi des codes-barres, des sites d'amorçage d'amplification et des sites d'amorçage de séquençage universels générant ainsi une bibliothèque de molécules d'acides nucléiques cibles concaténées.

6. Utilisation d'un kit de production d'une bibliothèque de molécules d'acides nucléiques cibles concaténées pour un procédé selon les revendications 1 à 4, ledit kit comprenant : un premier adaptateur ayant au moins une région bicaténaire, un second adaptateur comprenant un ou plusieurs parmi des codes-barres, des sites d'amorçage d'amplification et des sites d'amorçage de séquençage universels, une exonucléase, une polymérase d'acide nucléique et une ligase d'acide nucléique, et comprenant éventuellement en outre des amorces d'amplification complémentaires des séquences du premier adaptateur, une polymérase d'acide nucléique thermostable et un mélange d'au moins quatre désoxynucléoside triphosphates.
